# EUROPÄISCHE PATENTSCHRIFT

(11) **EP 2 881 070 B1**
(45) Veröffentlichungstag und Bekanntmachung des Hinweises auf die Patenterteilung: **19.09.2018**
(21) Anmeldenummer: 14004409.0
(22) Anmeldetag: 25.04.2013
(51) Int. Cl.: A61B 34/30, A61B 46/10, A61B 34/00, A61B 90/00, A61B 17/00

(54) **Chirurgierobotersystem und chirurgisches Instrument**
Surgical robot system and surgical instrument
Système robotisé chirurgical et instrument chirurgical

(30) Priorität: 27.04.2012 DE 102012008537; 15.03.2013 DE 102013004591
(43) Veröffentlichungstag der Anmeldung: 10.06.2015
(62) Teilanmeldung aus: 13719025.2
(73) Patentinhaber: KUKA Deutschland GmbH, 86165 Augsburg (DE)
(72) Erfinder: Lohmeier, Sebastian, 80639 München (DE); Schober, Wolfgang, 86554 Pöttmes (DE)
(74) Vertreter: Schlotter, Alexander Carolus Paul

(56) Entgegenhaltungen:
- WO-A1-2010/127940
- US-A- 5 891 094
- US-A1- 2010 079 099
- US-B1- 6 436 107

## Beschreibung

Ein Aspekt der vorliegenden Erfindung betrifft ein Chirurgierobotersystem mit einem Roboter und einer daran befestigten Instrumentenanordnung, eine solche Instrumentenanordnung mit einer Antriebseinheit, einem Instrument und einer Instrumenten-Schnittstelle sowie eine solche Antriebseinheit, ein solches Instrument und eine solche Instrumenten-Schnittstelle, sowie eine Hülle, insbesondere einer solchen Instrumenten-Schnittstelle, und ein Verfahren zu deren Applizierung.

Chirurgische Instrumente sollen möglichst steril sein. Auf der anderen Seite sind Roboter, bedingt beispielsweise durch Schmiermittel, Abtrieb und dergleichen, nur schwer sterilisierbar.

Aus der WO 2009/061915 A2 ist daher ein Roboter mit einer Adapteraufnahme bekannt, an der ein Adapter einer sterilen Hülle befestigt ist, die den Roboter umhüllt. Auf der sterilen, roboterabgewandten Seite ist ein Instrument befestigt, dessen Endeffektor durch Seilzüge im Instrumentenschaft aktuiert wird.

Hierzu sind Scheiben, die in dem sterilen Adapter nebeneinander drehbar gelagert sind, mit Gegenscheiben gekoppelt, die in den Roboterarm integriert sind. Die Drehantriebe zur Aktuierung der Gegenscheiben sind in der Roboterbasis angeordnet, die Antriebsmomente werden durch Seilzüge in dem Roboterarm übertragen, so dass das antriebslose Instrument leicht handhabbar ist.

Die WO 2010/127940 A1 betrifft ein steriles Abdecksystem zum sterilen Verkleiden eines medizintechnischen Roboterarms sowie Verfahren zum sterilen Verkleiden eines medizintechnischen Roboterarms. Das sterile Abdecksystem zum sterilen Verkleiden des medizintechnischen Roboterarms mit einem eine Aufnahmebohrung mit Innenwandung aufweisenden Anschlusselement für endoskopische Geräte ist versehen mit einer sackförmigen Drape zum Umhüllen des Anschlusselements. Die Drape weist einen an die Aufnahmebohrung angepassten Folienschlauch zur Abdeckung zur Abdeckung der Innenwandung und ein an einem ersten Ende des Folienschlauches befestigtes, abnehmbares Führungselement, das an die Aufnahmebohrung angepasst ist und im Ausgangszustand des sterilen Abdecksystems in den Folienschlauch hineinragt, auf. Der Folienschlauch ist mit dem Führungselement in die Aufnahmebohrung einführbar und durchführbar, derart, dass eine im Ausgangszustand des sterilen Abdecksystems nach innen gewandte Oberfläche des Folienschlauches im durch die Aufnahmebohrung durchgeführten Zustand des Folienschlauches der Innenwandung der Aufnahmebohrung zugewandt ist.

Eine Aufgabe eines Aspekts der vorliegenden Erfindung ist es, ein verbessertes Chirurgierobotersystem zur Verfügung zu stellen.

Diese Aufgabe wird durch eine Hülle mit den Merkmalen des Anspruchs 1 bzw. ein Verfahren zu deren Verwendung nach Anspruch 16 gelöst. Die Unteransprüche betreffen vorteilhafte Weiterbildungen.

Ein weiterer Aspekt der vorliegenden Erfindung betrifft ein, insbesondere robotergeführtes, chirurgisches Instrument mit einem Instrumententeil, insbesondere einem Instrumentenschaft mit einem Endeffektor, sowie einem damit lösbar koppelbaren Instrumentenmodul, insbesondere einem Antriebsmodul zum Aktuieren des Endeffektors, sowie ein Verfahren zum Ankoppeln eines solchen Instrumententeils.

In der minimalinvasiven Chirurgie wird ein chirurgisches Instrument durch eine lokale Öffnung in einen Patienten eingeführt. Insbesondere, wenn das Instrument durch einen Roboter geführt wird, kann ein Endeffektor im Inneren des Patienten, beispielsweise eine Schere oder endoskopische Optik, durch einen extrakorporalen Antrieb, zum Beispiel einen Elektromotor, aktuiert werden, beispielsweise verschwenkt und/oder geschlossen. Insbesondere, um unterschiedliche Endeffektoren verwenden und/oder die Sterilitätsanforderungen bei einer Operation besser erfüllen zu können, ist es vorteilhaft, wenn Antrieb und Endeffektor voneinander lösbar sind.

Aus der WO 2009/061915 A2 ist ein chirurgisches Instrument mit einem Antriebsmodul und einem Instrumentenschaft bekannt. Drehbare Koppelelemente des Antriebsmoduls und damit fluchtende drehbare Gegenelemente des Instrumentenschafts werden über sterile Kupplungen formschlüssig in einer ausgezeichneten Orientierung angekoppelt.

Eine Aufgabe eines Aspekts der vorliegenden Erfindung ist es, das Ankoppeln eines Instrumententeils an ein Instrumentenmodul eines, insbesondere robotergeführten, chirurgischen Instruments zu verbessern.

### Chirurgierobotersystem

Ein Chirurgierobotersystem nach einem Aspekt der vorliegenden Erfindung umfasst einen oder mehrere Roboter. Ein Roboter kann in einer Ausführung sechs oder mehr Gelenke, insbesondere Drehgelenke, aufweisen, wobei mehr als sechs Gelenke eine vorteilhafte Positionierung des redundanten Roboters ermöglichen können. Der bzw. die Roboter weisen in einer Ausführung eine Steuerung auf. Dabei können mehrere Roboter eine gemeinsame Zentralsteuerung oder Einzelsteuerungen aufweisen, die zur kompakteren Darstellung gemeinsam ebenfalls als eine Steuerung der Roboter bezeichnet wird. Ein Roboter kann in einer Ausführung an einem Operationstisch angeordnet, insbesondere lösbar befestigt, sein.

An einem oder mehreren Robotern des Chirurgierobotersystems ist jeweils eine Instrumentenanordnung nach einem nachfolgend erläuterten Aspekt der vorliegenden Erfindung befestigt. In einer Ausführung ist eine Instrumentenanordnung lösbar an einem Roboter befestigt, insbesondere formschlüssig, reibschlüssig und/oder magnetisch, insbesondere elektromagnetisch. In einer Ausführung weist die Instrumentenanordnung, insbesondere eine Antriebseinheit der Instrumentenanordnung, ein Gehäuse auf, welches an einer Außenseite des Roboters, insbesondere einem Roboter(end- bzw. -werkzeug)flansch, befestigt, beispielsweise verschraubt, verrastet oder geklemmt, ist. Zusätzlich oder alternativ kann insbesondere eine Instrumenten-Schnittstelle und/oder ein Instrument der Instrumentenanordnung an der Außenseite des Roboters, insbesondere einem Roboter(end- bzw. -werkzeug)flansch, befestigt sein.

### Instrumentenanordnung

Eine Instrumentenanordnung nach einem Aspekt der vorliegenden Erfindung ist entsprechend zur Befestigung an einem Roboter eingerichtet bzw. als robotergeführte Instrumentenanordnung ausgebildet. Sie umfasst eine Antriebseinheit, ein Instrument und eine Instrumenten-Schnittstelle nach einem der nachfolgend erläuterten Aspekte der vorliegenden Erfindung.

Indem die, vorzugsweise als Ganzes tragbar ausgebildete, Instrumentenanordnung selber die Antriebseinheit aufweist, kann vorteilhaft eine Übertragung von Antriebskräften von dem Roboter selber auf das Instrument entfallen, der Roboter daher kleiner bauen, was insbesondere die Kooperation mehrerer schlanker Roboter in einem begrenzten Operationsgebiet ermöglichen kann. Zudem kann die Antriebseinheit vorteilhafterweise einfach an unterschiedliche Instrumente angepasst oder auch ausgewechselt werden. Sie ist vorzugsweise als eigenständiges bzw. vom Roboter unabhängiges Modul ausgebildet.

In einer Ausführung umfasst eine Instrumentenanordnung zwei oder mehr verschiedene Antriebseinheiten und/oder zwei oder mehr verschiedene Instrumente, die wahlweise, insbesondere modulartig, mit einem Instrument bzw. einer Antriebseinheit zu einer an dem Roboter befestigten Instrumentenanordnung verbindbar sind. Verschiedene Antriebseinheiten bzw. Instrumente können sich insbesondere in der Anzahl und/oder Leistung aktuierter Freiheitsgrade unterscheiden. So kann beispielsweise eine Antriebseinheit mit drei aktuierten Freiheitsgraden wahlweise mit einem Instrument mit einem oder zwei aktuierten Freiheitsgraden und zwei bzw. einem nicht aktuierten, d.h. ungenutzen bzw. blinden Freiheitsgrad, und mit Instrumenten mit drei aktuierten Freiheitsgraden verbunden werden, die unterschiedliche Endeffektoren aufweisen.

Instrument und Antriebseinheit sind lösbar miteinander verbunden, wobei zwischen Antriebseinheit und Instrument eine Instrumenten-Schnittstelle angeordnet ist. Instrument und Antriebseinheit können insbesondere form-, reib-, stoffschlüssig und/oder magnetisch, vorzugsweise elektromagnetisch aneinander und/oder an der dazwischen angeordneten Instrumenten-Schnittstelle befestigt sein. Insbesondere kann die Instrumenten-Schnittstelle mit der Antriebseinheit und/oder dem Instrument verschraubt, verrastet, geklemmt oder auch verklebt sein, wobei die Klebestelle als Soll-Trennstelle ausgebildet ist. Zusätzlich oder alternativ kann die Antriebseinheit mit dem Instrument verschraubt, verrastet oder geklemmt sein. In einer Ausführung weist die Instrumenten-Schnittstelle einen formstabilen Flansch zur Befestigung der Antriebseinheit und/oder des Instruments auf. Die Antriebseinheit ist in einer Ausführung an einem proximalen bzw. endeffektorfernen Ende des Instruments angeordnet.

### Antriebseinheit

Eine Antriebseinheit nach einem Aspekt der vorliegenden Erfindung weist einen oder mehrere, insbesondere drei oder vier, Drehantriebe mit je wenigstens einer Antriebswelle auf. In einer Ausführung weisen ein oder mehrere Drehantriebe der Antriebseinheit je einen oder mehrere Elektromotoren, insbesondere Gleich- oder Wechselstrommotoren, mit einem Stator und einem Rotor auf. Zusätzlich oder alternativ können ein oder mehrere Drehantriebe der Antriebseinheit je einen oder mehrere Hydraulikmotoren und/oder Piezo-Antriebe aufweisen, die die jeweiligen Antriebswelle(n) antreiben bzw. mit einem Drehmoment beaufschlagen können. Die Antriebswelle kann insbesondere ein Rotor bzw. Läufer eines Elektro- und/oder Hydraulikmotors sein. In einer Ausführung weist ein Drehantrieb ein, vorzugsweise koaxiales, Getriebe, insbesondere ein Umlaufgetriebe, vorzugsweise ein Planeten- oder Harmonic-Drive-Getriebe auf, wobei die Antriebswelle eine Abtriebs- bzw. Ausgangswelle des Getriebes sein kann. In einer anderen Ausführung ist ein Drehantrieb als Direktantrieb ausgebildet. Hierunter wird vorliegend insbesondere verstanden, dass die Antriebswelle direkt durch ein, insbesondere hydraulisch, elektrisch und/oder (elektro)magnetisch erzeugtes, Antriebsdrehmoment ohne zwischengeschaltetes Getriebe beaufschlagt wird. Insbesondere ein solcher Direktantrieb kann in einer Ausführung selbsthemmungsfrei ausgebildet sein. Hierdurch kann vorteilhaft das Instrument mit angeflanschter Antriebseinheit aus einem Patienten entfernt werden. Zusätzlich oder alternativ kann ein Drehantrieb einen, insbesondere koaxialen, Sensor, insbesondere einen an- und/oder abtriebsseitigen Drehzahl- und/oder Drehmomentsensor aufweisen.

Die Antriebswelle weist ein Koppelteil zur Koppelung mit einer Antriebswelle des Instruments auf, das drehfest mit der Antriebswelle ist. Es kann in einer Ausführung axialfest mit der Antriebswelle, insbesondere integral mit dieser, ausgebildet sein. In einer anderen Ausführung ist das Koppelteil axial verschieblich an der Antriebswelle angeordnet, insbesondere formschlüssig, beispielsweise mittels einer Passfeder, einer Keilverzahnung, eines Zahn- oder Polygonwellenprofils oder dergleichen. In einer Weiterbildung ist das Koppelteil axial vorgespannt, insbesondere durch ein Federmittel gegen die an der Antriebseinheit befestigte Instrumenten-Schnittstelle oder das an der Antriebseinheit befestigte Instrument. Hierdurch kann insbesondere eine Axialtoleranz kompensiert und/oder eine Anpresskraft für eine reibschlüssige Verbindung dargestellt werden.

Eine oder mehrere Antriebswellen sind in einer Ausführung als Hohlwellen ausgebildet. In einer Ausführung weist die Antriebseinheit eine oder mehrere Antriebswellen auf, die jeweils konzentrisch bzw. koaxial in einer sie umgebenden Antriebs-Hohlwelle angeordnet, insbesondere gelagert sind. Zur kompakteren Darstellung wird dabei jeweils die Antriebswelle, die in einer anderen Antriebswelle angeordnet ist, als innere Antriebswelle, die andere Antriebswelle als äußere Antriebswelle bezeichnet. Weist beispielsweise die Antriebseinheit in einer Ausführung drei Antriebswellen auf, so bildet die innerste Antriebswelle, die ebenfalls als Antriebs-Hohlwelle ausgebildet sein kann, eine innere Antriebswelle, die sie umgebende mittlere Antriebswelle eine äußere Antriebswelle und zugleich eine (weitere) innere Antriebswelle, die sie umgebende äußerste Antriebswelle eine (weitere) äußere Antriebswelle. In gleicher Weise kann die Antriebseinheit auch vier oder mehr konzentrische Antriebs-Hohlwellen aufweisen.

In einer Ausführung sind ein oder mehrere Drehantriebe koaxial zu ihrer jeweiligen Antriebswelle, insbesondere einer gemeinsamen Drehachse koaxialer Antriebswellen angeordnet. Insbesondere können ein oder mehrere Drehantriebe fluchtend hintereinander angeordnet sein. Gleichermaßen können ein oder mehrere Drehantriebe parallel in radialer und/oder Umfangsrichtung versetzt zu ihren Antriebswellen, insbesondere einer gemeinsamen Drehachse koaxialer Antriebswellen angeordnet und mit diesen insbesondere über ein Stirn- oder Reibradgetriebe gekoppelt sein. Gleichermaßen können ein oder mehrere Drehantriebe winkelig, insbesondere rechtwinkelig, zu ihrer jeweiligen Antriebswelle, insbesondere einer gemeinsamen Drehachse koaxialer Antriebswellen angeordnet und mit diesen insbesondere über ein Schnecken-, Schrauben- oder Kronenradgetriebe gekoppelt sein.

Die Antriebseinheit kann jeweils drahtlos oder leitungsgebunden mit einer Energiequelle und/oder der Steuerung verbunden sein. In einer Ausführung umhüllt die nachfolgend erläuterte Hülle der Instrumenten-Schnittstelle auch, wenigstens teilweise, eine Energie- und/oder Signalleitung der Antriebseinheit.

### Instrument

Ein Instrument nach einem Aspekt der vorliegenden Erfindung weist einen Instrumentenschaft auf, an dessen distalem bzw. antriebseinheitsabgewandten Ende ein ein- oder mehrteiliger Endeffektor angeordnet sein kann, insbesondere ein Skalpell, Zangen- bzw. Scherenschenkel oder dergleichen.

Das Instrument ist in einer Ausführung ein Endo- bzw. minimal-invasives chirurgisches Instrument ("MIC"), insbesondere ein endoskopisches, beispielsweise laparoskopisches oder thorakoskopisches. Insbesondere kann der Instrumentenschaft dazu vorgesehen bzw. eingerichtet sein, durch einen Eintritt, der vorzugsweise im Wesentlichen dem Außendurchmesser des Instrumentenschaftes entspricht, insbesondere durch einen Trokar, in den Patienten eingeführt und dort aktuiert zu werden.

Der Endeffektor kann ein oder mehrere Freiheitsgrade aufweisen. Insbesondere können ein oder mehrere Teile des Endeffektors je ein oder zwei Drehfreiheitsgrade um Drehachsen aufweisen, die vorzugsweise senkrecht zur Schaftachse stehen. Beispielsweise kann ein zweiteiliger Endeffektor eine Zange bzw. Schere darstellen, deren Schenkel gegensinnig um dieselbe Drehachse verschwenken.

Zur Aktuierung des Endeffektors weist das Instrument eine oder mehrere Antriebswellen, insbesondere eine Antriebswelle zur Aktuierung jeden Freiheitsgrades, in einer Ausführung also insbesondere ein, zwei oder drei Antriebswellen auf.

Die Antriebswelle weist ein Koppelteil zur Koppelung mit einer Antriebswelle der Antriebseinheit auf, das drehfest mit der Antriebswelle ist. Es kann in einer Ausführung axialfest mit der Antriebswelle, insbesondere integral mit dieser ausgebildet, sein. In einer anderen Ausführung ist das Koppelteil axial verschieblich an der Antriebswelle angeordnet, insbesondere formschlüssig, beispielsweise mittels einer Passfeder, einer Keilverzahnung, eines Zahn- oder Polygonwellenprofils oder dergleichen. In einer Weiterbildung ist das Koppelteil axial vorgespannt, insbesondere durch ein Federmittel gegen die an dem Instrument befestigte Instrumenten-Schnittstelle oder die an dem Instrument befestigte Antriebseinheit.

Eine oder mehrere Antriebswellen sind in einer Ausführung als Hohlwellen ausgebildet. In einer Ausführung weist die Antriebseinheit eine oder mehrere Antriebswellen auf, die jeweils konzentrisch bzw. koaxial in einer sie umgebenden Antriebs-Hohlwelle angeordnet, insbesondere gelagert sind.

Indem die (innerste) Antriebswelle des Instruments als Antriebs-Hohlwelle ausgebildet ist, wird in einer Ausführung vorteilhaft ein Ein- bzw. Durchführen eines Zusatz-Instruments ermöglicht.

In einer Ausführung fluchten miteinander gekoppelte Antriebswellen von Antriebseinheit und Instrument miteinander. In einer Ausführung ist(sind) die Antriebswelle(n) koaxial, insbesondere fluchtend oder parallel versetzt zu dem Instrumentenschaft angeordnet. Hierdurch kann in einer Ausführung eine radial kompakte Bauform erreicht werden. In einer anderen Ausführung ist(sind) die Antriebswelle(n) winkelig, insbesondere rechtwinkelig, zu dem Instrumentenschaft angeordnet. Hierdurch kann in einer Ausführung die Antriebseinheit winkelig, insbesondere rechtwinkelig, zu dem Instrumentenschaft angeordnet werden.

In einer Ausführung weist das Instrument ein instrumentenseitiges Umsetzungsgetriebe zur Umsetzung einer Rotation einer oder mehrerer Antriebswellen in eine entsprechende Translation eines bzw. mehrerer Zug- und/oder Schubmittel auf. Ein Zugmittel kann insbesondere ein oder mehrere, insbesondere gegenläufige, Seil-, Riemen- oder Bandtrumme aufweisen, ein Zug- und/oder Schubmittel ein oder mehrere, insbesondere gegenläufige, Zug- und/oder Schubstangen.

In einer Ausführung weist das Umsetzungsgetriebe eine, insbesondere für eine oder mehrere Antriebswellen der Antriebseinheit je eine, Kulissenführung auf. Eine Kulissenführung umfasst nach einer Ausführung eine Schiebehülse, die relativ zum Instrumentenschaft drehfest und axial verschieblich gelagert ist, insbesondere an dem Instrumentenschaft oder einer umgebenden Antriebshohlwelle. In einer von der Schiebehülse und der Antriebswelle ist eine, insbesondere gegen die Axialrichtung geneigte, Kulisse bzw. Nut ausgebildet, in der ein Passelement, insbesondere eine Passfeder, von dem anderen von der Schiebehülse und der Antriebswelle formschlüssig geführt ist. Auf diese Weise wird eine Drehung der Antriebswelle in eine axiale Translation der Schiebehülse umgesetzt, die so insbesondere ein Zug- und/oder Schubmittel aktuieren kann.

Wenn in einer Ausführung eine Antriebswelle in einer Antriebshohlwelle gelagert ist, kann in einer Weiterbildung eine mit einer Antriebswelle gekoppelte Schiebehülse einer Kulissenführung zugleich ein Radiallager, insbesondere ein Loslager, zwischen dieser und einer benachbarten Antriebswelle bilden. Ein Festlager einer Antriebswelle ist vorzugsweise an einem antriebseinheitszugewandten bzw. proximalen Ende des Instrumentenschaftes angeordnet

In einer Ausführung ist das Umsetzungsgetriebe in einer antriebseinheitszugewandten bzw. proximalen Hälfte des Instrumentenschaftes angeordnet. In einer anderen Ausführung ist das Umsetzungsgetriebe in einer antriebseinheitsabgewandten bzw. distalen Hälfte des Instrumentenschaftes angeordnet. Hierdurch kann vorteilhafterweise die Aktuierung über einen großen Bereich des Instrumentenschaftes durch die Zug- und/oder Schubmittel bzw. durch die Antriebswellen übertragen werden.

### Zusatz-Instrument

Nach einem Aspekt der vorliegenden Erfindung weist eine Instrumentenanordnung ein Zusatz-Instrument auf, welches, insbesondere lösbar, durch das Instrument der Instrumentenanordnung, insbesondere ein Führungsrohr des Instruments ein- bzw. durchgeführt, insbesondere mit Radialspiel oder einer Radialpassung eingesteckt, ist. Hierzu kann das Zusatz-Instrument in einer Ausführung rohrförmig ausgebildet und starr oder flexibel sein. Das Instrument kann entsprechend in einer Ausführung ein, insbesondere starres, Führungsrohr aufweisen, welches, insbesondere zentrisch, in dem Instrumentenschaft angeordnet sein und/oder sich - wenigstens im Wesentlichen - über die gesamte Innenlänge des Instrumentenschaftes erstrecken kann. In einer Weiterbildung kann eine (innerste) Antriebshohlwelle des Instruments als Führungsrohr fungieren bzw. ein Führungsrohr bilden.

Das Zusatz-Instrument kann in einer Ausführung als Führung für gasförmige und/oder flüssige Medien, insbesondere als Saug- und/oder Zufuhrpassage, und/oder als elektrischer und/oder Lichtwellenleiter ausgebildet sein, um beispielsweise Spül- oder Wasserstrahlchirurgie-Medien, insbesondere unter Unter- bzw. Überdruck, ab- bzw. zuzuführen, Laser- und/oder Beleuchtungslicht und/oder Strom in den Patienten zu leiten und/oder optische und/oder elektrische Signale aus ihm herauszuführen.

In einer Ausführung kann das Zusatz-Instrument auf einer der Antriebseinheit abgewandten Seite der Instrumentenschnittstelle durch das Instrument eingeführt sein, so dass es vorteilhafterweise nicht gegen die Antriebseinheit steril abgegrenzt werden muss. In einer anderen Ausführung ist das Zusatz-Instrument durch die Antriebseinheit, insbesondere eine (innerste) Antriebshohlwelle der Antriebseinheit eingeführt.

Zusätzlich oder alternativ zu dem Zusatz-Instrument kann ein Antriebsmittel zum Betätigen eines Endeffektors, insbesondere lösbar, durch das Instrument der Instrumentenanordnung, insbesondere eine (innerste) Antriebshohlwelle des Instruments eingeführt, insbesondere mit Radialspiel oder einer Radialpassung eingesteckt, sein. Hierzu kann das Antriebsmittel in einer Ausführung rohrförmig ausgebildet und starr oder flexibel sein. Das Antriebsmittel kann insbesondere ein oder mehrere Zug- und/oder Schubmittel und/oder ein oder mehrere Drehwellen aufweisen.

### Instrumenten-Schnittstelle

Eine Instrumenten-Schnittstelle nach einem Aspekt der vorliegenden Erfindung weist eine die Antriebseinheit, insbesondere hermetisch bzw. steril, umschließende Hülle auf. Die Hülle ist in einer Ausführung flexibel, insbesondere folienartig. In einer Ausführung ist die Hülle auf der antriebseinheitsabgewandten Außenseite steril oder sterilisierbar.

Auf diese Weise kann die Antriebseinheit, die aufgrund von Abrieb, Schmiermittel, temperatur- und/oder feuchtigkeitsempfindlicher Komponenten oder dergleichen nur schwer sterilisierbar sein kann, gegen die Operationsumgebung steril abgeschirmt werden, wobei die Aktuierung durch bzw. über die Schnittstelle in das, vorteilhafterweise einfach sterilisierbare, Instrument übertragen wird.

In einer Ausführung sind Koppelteile einer oder mehrerer Antriebswellen der Antriebseinheit und entsprechender, insbesondere koaxialer, Antriebswellen des Instruments magnetisch miteinander gekoppelt. Insbesondere in diesem Fall kann die Instrumenten-Schnittstelle durch eine einfache Folie gebildet werden, wobei vorzugsweise ein Luftspalt zwischen den magnetisch miteinander gekoppelten Koppelteilen ausgebildet ist.

In einer Ausführung weist die Instrumenten-Schnittstelle ein oder mehrere drehbare Zwischenelemente auf, die dazu eingerichtet sind, mit je einem Koppelteil einer Antriebswelle der Antriebseinheit und einem Koppelteil einer Antriebswelle des Instruments, insbesondere reib- oder formschlüssig, gekoppelt zu sein, wenn die Schnittstelle zwischen Antriebseinheit und Instrument angeordnet, insbesondere an Antriebseinheit und/oder Instrument befestigt ist.

Die Anordnung der Zwischenelemente entspricht vorzugsweise der Anordnung der Antriebswellen bzw. ihrer Koppelteile. Sind also in einer Ausführung die Antriebswellen von Antriebseinheit und/oder Instrument konzentrisch, so sind insbesondere auch die Zwischenelemente konzentrisch zueinander angeordnet, wobei vorzugsweise, wie vorstehend erläutert, jeweils ein, insbesondere ringförmiges, inneres Zwischenelement, konzentrisch in einem äußeren ringförmigen Zwischenelement angeordnet, insbesondere gelagert ist. In einer Weiterbildung sind die Zwischenelemente abgedichtet gelagert, beispielsweise durch Lagerringe, die Labyrinthdichtungen aufweisen oder dergleichen. Unter abgedichtet wird dabei vorliegend insbesondere steril im medizinischen Sinne verstanden, insbesondere derart abgedichtet, dass feste, vorzugsweise auch flüssige, insbesondere auch gasförmige Elemente einer vorgegebenen Größe die Dichtung höchstens in einer vorgegebenen Höchstmenge bzw. -rate, die auch gleich Null sein kann, überwinden können.

Die Instrumenten-Schnittstelle kann einen formstabilen Flansch zur Befestigung der Antriebseinheit und/oder des Instruments aufweisen, in dem die Zwischenelemente, insbesondere abgedichtet, drehbar gelagert sind.

Zur reibschlüssigen Koppelung können Zwischenelemente und Koppelteile von Antriebseinheit und/oder Instrument Kontaktflächen aufweisen, die einander berühren, wenn die Instrumenten-Schnittstelle zwischen Antriebseinheit und Instrument angeordnet ist und Instrument und Antriebseinheit direkt und/oder über die Instrumenten-Schnittstelle verbunden sind. Zur formschlüssigen Koppelung können solche Kontaktflächen ineinander eingreifende Vorsprünge und Aussparungen bzw. komplementäre Absätze aufweisen. Insbesondere können an einem von einem Koppelteil und einem Zwischenelement ein oder mehrere Vorsprünge bzw. Absätze angeordnet sein, die in Aussparungen bzw. komplementäre Absätze in dem anderen von dem Koppelteil und dem Zwischenelement formschlüssig eingreifen, wenn die Instrumenten-Schnittstelle, insbesondere ein formstabiler Flansch der Instrumenten-Schnittstelle, an der Antriebseinheit bzw. dem Instrument angeordnet ist. Vorzugsweise können Koppelteil und Zwischenelement miteinander kämmende Stirn-, insbesondere Hirth-Verzahnungen aufweisen.

Die Reib- oder Formschluss-Kontaktflächen können in einer Ausführung kegelförmig ausgebildet sein. Hierdurch kann vorteilhaft eine Selbstzentrierung und/oder, insbesondere in Kombination mit axial verschieblichen, vorzugsweise vorgespannten, Koppelteilen, eine Kompensation einer Axialtoleranz erreicht werden.

In einer Ausführung weist die Hülle eine Innendurchführung für ein durch das Instrument und die Antriebseinheit der Instrumentenanordnung eingeführtes Zusatz-Instrument auf. Diese Innendurchführung kann schlauchartig ausgebildet sein und eine inner(st)e Antriebshohlwelle der Antriebseinheit durchgreifen. In einer Weiterbildung ist sie abgedichtet, insbesondere drehbar, mit einem inner(st)en, mit der Antriebshohlwelle gekoppelten Zwischenelement der Instrumenten-Schnittstelle verbunden.

In einer Weiterbildung weist die Innendurchführung einen Blindstopfen und einen Abschlussring auf. In einem Ausgangs- bzw. Montagezustand ist der Blindstopfen an einem Ende der Innendurchführung befestigt, verschließt diese und deckt einen Umfangsbereich der Innendurchführung ab. Der Blindstopfen kann dann durch die Antriebseinheit gezogen werden, so dass er durch eine Austrittsöffnung der Hülle aus dieser austritt und entfernt werden kann. Anschließend kann der Abschlussring an dem Umfangsbereich der Innendurchführung befestigt werden, der durch Entfernen des Blindstopfens freigelegt wurde, und zudem die Austrittsöffnung der Hülle verschließen. Auf diese Weise kann eine torusartige Hülle mit einer sterilen Außenoberfläche zur Verfügung gestellt werden, in deren Ringraum die Antriebseinheit angeordnet und so steril gegen die OP-Umgebung abgegrenzt ist, und deren Durchgangsöffnung zur Einführung des Zusatz-Instruments zur Verfügung steht.

Die vorstehend erläuterte Weiterbildung eignet sich insbesondere für die vorstehend erläuterte Antriebseinheit mit einer Antriebs-Hohlwelle. Sie kann gleichermaßen zum, insbesondere sterilen, Umhüllen eines Roboters mit einem Werkzeugflansch verwendet werden, der eine Hohlwelle aufweist. Unter einem (sterilen) Umhüllen wird vorliegend insbesondere ein teilweises oder vollständiges bzw. allseitig geschlossenes, insbesondere hermetisches, Abdecken bzw. Umschließen verstanden.

Nach einem Aspekt der vorliegenden Erfindung weist daher eine Hülle, die insbesondere eine oder mehrere Merkmale der vorstehend beschriebenen Hülle der Instrumenten-Schnittstelle aufweisen kann, allgemein eine schlauchartige Innendurchführung zum Durchführen durch eine Hohlwelle eines Roboters oder einer vorstehend erläuterten Antriebseinheit und durch eine Austrittsöffnung der Hülle auf, wobei die Innendurchführung einen Blindstopfen und einen ein- oder mehrteiligen Abschlussring zur Befestigung an der Austrittsöffnung und einen, insbesondere äußeren, Umfangs- bzw. Mantelflächenbereich der Innendurchführung aufweist, der durch Entfernen des Blindstopfens frei wird. Die Innendurchführung kann insbesondere drehbar an der Hülle gelagert oder integral mit dieser ausgebildet sein. Sie kann insbesondere starr oder flexibel ausgebildet sein. Zur kompakteren Darstellung wird auch eine starre rohrförmige Innendurchführung verallgemeinernd als schlauchartig bezeichnet.

Zum Umhüllen des Roboters oder der Antriebseinheit wird zunächst die mit dem Blindstopfen versehene Innendurchführung durch die Hohlwelle und die Austrittsöffnung der Hülle durchgeführt. Der Blindstopfen, der vorzugsweise eine geschlossene Außenstirnseite und/oder eine rohrartige Mantelfläche aufweist, verhindert dabei eine Verschmutzung des Inneren der schlauchartigen Innendurchführung und des durch ihn abgedeckten Umfangsbereichs der Innendurchführung. Gleichermaßen kann auch die Innendurchführung zunächst stirnseitig verschlossen sein, wobei der Verschlussbereich anschließend abgetrennt wird. Um das Durchführen zu erleichtern, kann der Blindstopfen eine starre oder flexible Einführhilfe, insbesondere eine Schnur oder einen Stab, aufweisen.

Anschließend wird der Blindstopfen entfernt und der Abschlussring an dem Umfangsbereich der Innendurchführung befestigt, der durch das Entfernen des Blindstopfens frei geworden ist. Wie vorstehend beschrieben, kann der Abschlussring, der starr oder flexibel ausgebildet sein kann, in einer Weiterbildung an der Austrittsöffnung der Hülle befestigt werden und diese - bis auf die Innendurchführung - verschließen. Ein an dem Umfangsbereich der Innendurchführung befestigter Teil des Abschlussringes kann insbesondere drehbar an einem an der Austrittsöffnung der Hülle befestigten Teil des Abschlussringes gelagert oder integral bzw. einteilig mit diesem ausgebildet sein.

Der Umfangsbereich der Innendurchführung, der durch das Entfernen des Blindstopfens frei geworden ist, wurde durch den Blindstopfen vor Verschmutzung beim Durchführen geschützt. Unter einem Befestigen an dem Umfangsbereich wird vorliegend insbesondere ein Befestigen derart, dass der Umfangsbereich in Längsrichtung der Innendurchführung gesehen ganz oder teilweise von dem Abschlussring abgedeckt ist, verstanden. Der Umfangsbereich, der durch das Entfernen des Blindstopfens frei geworden ist, kann in Längsrichtung ein- oder beidseitig über den Abschlussring hinausragen. Gleichermaßen kann der Abschlussring außer dem Umfangsbereich, der durch das Entfernen des Blindstopfens frei geworden ist, oder einem Teil dieses Umfangsbereichs auch einen Umfangsbereich der Innendurchführung abdecken, der zuvor nicht von dem Blindstopfen abgedeckt war.

Nach einem Aspekt der vorliegenden Erfindung weist ein chirurgisches Instrument ein Instrumentenmodul und ein damit lösbar verbindbares, insbesondere verbundenes, Instrumententeil auf. Das chirurgische Instrument kann insbesondere robotergeführt sein bzw. das Instrumentenmodul oder Instrumententeil eine, insbesondere elektromechanische, Schnittstellung zur, insbesondere mechanischen und/oder signaltechnischen, Befestigung an einem Roboter aufweisen. Nach einem Aspekt wird ein Roboter mit einem robotergeführten chirurgischen Instrument unter Schutz gestellt. In einer Ausführung ist das chirurgische Instrument ein minimalinvasives Instrument, das zum teilweisen Einführen in einen Patienten durch eine sogenannte Trokaröffnung vorgesehen bzw. eingerichtet ist.

Das Instrumentenmodul weist in einer Ausführung einen in einen Patienten einführbaren Instrumentenschaft mit einem Endeffektor auf, das damit lösbar verbindbare Instrumententeil einen Antrieb zum Aktuieren des Endeffektors. Gleichermaßen kann auch das Instrumentenmodul einen Antrieb zum Aktuieren eines Endeffektors eines in einen Patienten einführbaren Instrumentenschafts des Instrumententeils aufweisen. Zur kompakteren Darstellung wird daher vorliegend allgemein von Instrumentenmodul und -teil gesprochen, das jeweils als Antriebs- bzw. Endeffektormodul bzw. -teil ausgebildet sein kann.

Der Endeffektor kann insbesondere ein Skalpell, eine Sonde, Schere, Zange oder Klemme, eine Optik zum Aussenden und/oder Empfangen elektromagnetischer Strahlung und/oder eine Fluidöffnung zum Ausführen und/oder Einsaugen von Gas und/oder Flüssigkeit sein. Ein Aktuieren des Endeffektors kann insbesondere ein Verschwenken des Endeffektors um eine, zwei oder drei Achsen relativ zu dem Instrumentenschaft und/oder ein Betätigen, insbesondere Öffnen bzw. Schließen, des Endeffektors umfassen, insbesondere sein. Der Antrieb kann einen oder mehrere Elektromotoren aufweisen. Zusätzlich oder alternativ kann der Antrieb ein oder mehrere Handelemente, insbesondere Griffe und/oder Räder, zum manuellen Aktuieren des Endeffektors aufweisen. Allgemein kann der Antrieb zum elektromotorischen, elektromagnetischen, pneumatischen, hydraulischen und/oder manuellen Aktuieren des Endeffektors eingerichtet sein.

Um eine Bewegung bzw. Kraft zwischen Antrieb und Endeffektor zu vermitteln, wobei auch ein antiparalleles Kräftepaar, d.h. ein Drehmoment verallgemeinernd eine Kraft im Sinne der vorliegenden Erfindung sein kann, weist das Instrumentenmodul eine Koppelelementanordnung mit einem oder mehreren Koppelelementen auf, das damit lösbar verbindbare Instrumententeil eine Gegenelementanordnung mit einem oder mehreren Gegenelementen zum Ankoppeln der Koppelelementanordnung.

Ein oder mehrere Koppelelemente und das bzw. die damit koppelbaren bzw. gekoppelten Gegenelemente sind in einer Ausführung translatorisch beweglich, um jeweils einen Freiheitsgrad des Endeffektors zu aktuieren. Insbesondere können solche Koppelelemente in einem Schublager des Instrumentenmoduls, vorzugsweise verdrehsicher, verschiebbar geführt, insbesondere als Stößel ausgebildet sein. Zusätzlich oder alternativ können ein oder mehrere Koppelelemente und das bzw. die damit koppelbaren bzw. gekoppelten Gegenelemente rotatorisch beweglich sein, um jeweils einen Freiheitsgrad des Endeffektors zu aktuieren. Insbesondere können solche Koppelelemente in einem Drehlager des Instrumentenmoduls, vorzugsweise axialfest, verdrehbar geführt, insbesondere als Welle ausgebildet sein.

Nach einem Aspekt der vorliegenden Erfindung sind ein oder mehrere, insbesondere alle Koppelelemente der Koppelelementanordnung mit der Gegenelementanordnung bzw. deren Gegenelement(en) magnetisch koppelbar bzw. gekoppelt. Hierzu weisen in einer Ausführung das bzw. die Koppelelemente der Koppelelementanordnung des Instrumentenmoduls jeweils eine Magnetanordnung zum magnetischen Ankoppeln eines Gegenelements der Gegenelementanordnung auf. Das bzw. die Gegenelemente des Instrumententeils weisen in einer Ausführung entsprechend einen magnetisch beaufschlagbaren Bereich auf. Unter einem magnetisch beaufschlagbaren Bereich wird vorliegend insbesondere ein Bereich aus einem Material verstanden, das eine Permeabilitätszahl bzw. relative Permeabilität µᵣ aufweist, die wenigstens 10 beträgt, insbesondere einen Bereich aus einem ferromagnetischen oder dauermagnetischen Material.

Durch die magnetische Ankopplung der Koppel- und Gegenelementanordnung kann der Antrieb auf vorteilhafte Weise, insbesondere einfach, steril und/oder zuverlässig, lösbar mit dem Endeffektor wirkverbunden werden. Dabei kann die Magnetanordnung in einer Ausführung antriebsseitig angeordnet bzw. das Instrumentenmodul als Antriebsmodul ausgebildet sein. Dadurch kann insbesondere das Instrumententeil mit Instrumentenschaft und Endeffektor leichter, kompakter und/oder billiger, insbesondere als Einmalartikel, ausgebildet und/oder besser sterilisierbar werden bzw. sein. Gleichermaßen kann die Magnetanordnung auch endeffektorseitig angeordnet sein bzw. das Instrumentenmodul den Instrumentenschaft und Endeffektor aufweisen.

In einer Ausführung kann die Magnetanordnung eines oder mehrerer Koppelelemente einen oder mehrere Permanent- bzw. Dauermagnete aufweisen.

In einer Ausführung sind der bzw. die Permanentmagnete magnetisch derart dimensioniert, dass sie das jeweilige Gegenelement betriebssicher ankoppeln, wenn es dem Koppelelement benachbart ist, jedoch durch eine entsprechende größere Demontagekraft von diesem abgekoppelt werden können, insbesondere, indem Koppel- und Gegenelement voneinander beabstandet werden.

Vorzugsweise können jedoch ein Koppel- und ein Gegenelement voneinander abgekoppelt werden, ohne sie voneinander zu beabstanden.

Hierzu weist die Magnetanordnung eines oder mehrerer Koppelelemente in einer Ausführung einen oder mehrere Elektromagnete auf, die, insbesondere durch ein hierzu eingerichtetes Steuermittel, das insbesondere in einer Antriebssteuerung des Instruments implementiert sein kann, wahlweise bestrombar ist bzw. bestromt wird.

Durch wahlweise bestrombare Elektromagnete kann in einer Ausführung eine stromlos geöffnete Ankopplung zwischen Koppel- und Gegenelement bzw. ein sogenanntes Ruhestromprinzip vorgesehen sein. Dies kann es vorzugsweise ermöglichen, Instrumentenmodul und -teil bzw. Antrieb und Endeffektor stromlos und somit zuverlässig zu trennen, um beispielsweise auch bei einem Defekt den Endeffektor manuell aus dem Patienten entfernen zu können.

Gleichermaßen kann durch wahlweise bestrombare Elektromagnete in einer Ausführung eine stromlos geschlossene Ankopplung zwischen Koppel- und Gegenelement bzw. ein sogenanntes Arbeitsstromprinzip vorgesehen sein. Dies kann es vorzugsweise ermöglichen, auch bei einem Stromausfall Instrumentenmodul und -teil bzw. Antrieb und Endeffektor zuverlässig zu koppeln.

In einer Ausführung weist eine Magnetanordnung hierzu sowohl einen oder mehrere wahlweise bestrombare Elektromagnete als auch einen oder mehrere hierzu gegensinnige Permanentmagnete auf. Unter einem zu einem Elektromagneten gegensinnigen Permanentmagnet wird vorliegend insbesondere ein Permanentmagnet verstanden, dessen Magnetfeld durch den bestromten Elektromagneten in einem Koppelbereich von Koppel- und Gegenelement geschwächt, insbesondere - wenigstens im Wesentlichen - kompensiert bzw. neutralisiert wird. Fällt diese Schwächung bzw. Kompensation bei unbestromtem Elektromagneten fort, koppelt der dann ungeschwächte bzw. -kompensierte Permanentmagnet Koppel- und Gegenelement aneinander.

Zusätzlich oder alternativ kann in einer Ausführung eine Magnetanordnung sowohl einen oder mehrere wahlweise bestrombare Elektromagnete als auch einen oder mehrere hierzu gleichsinnige Permanentmagnete aufweisen. Unter einem zu einem Elektromagneten gleichsinnigen Permanentmagnet wird vorliegend insbesondere ein Permanentmagnet verstanden, dessen Magnetfeld durch den bestromten Elektromagneten in einem Koppelbereich von Koppel- und Gegenelement verstärkt wird. Hierdurch kann vorteilhaft die Haftkraft erhöht werden. Gegenüber einer Lösung ohne Permanentmagnet kann in einer Ausführung der zur Kraftübertragung erforderliche Elektromagnetstrom und damit der Energieverbrauch und die Wärmeentwicklung reduziert werden. Gegenüber einer Lösung ohne Elektromagnet kann in einer Ausführung der Permanentmagnet reduziert werden. Zusätzlich oder alternativ können in einer Ausführung ein oder mehrere Permanentmagnete einer Magnetanordnung an, insbesondere in, dem Koppelelement zwischen einer Verriegelungs- und einer hiervon verschiedenen Entriegelungsposition verstellbar, insbesondere verschieb- und/oder -drehbar gelagert sein bzw. verstellt, insbesondere verschoben und/oder -dreht werden. In einer Weiterbildung sind der bzw. die Permanentmagnete elektromotorisch, hydraulisch, pneumatisch und/oder manuell verstellbar. Zusätzlich oder alternativ können der bzw. die Permanentmagnete in der Verriegelungs-, der Entriegelungs- und/oder einer von beiden verschiedenen weiteren Position arretierbar sein.

Indem in einer Ausführung ein Permanentmagnet von einem Koppelbereich von Koppel- und Gegenelement entfernt wird, kann die magnetische Kopplung von Koppel- und Gegenelement durch diesen Permanentmagnet reduziert und so Koppel- und Gegenelement entkoppelt werden. Zusätzlich oder alternativ kann in einer Ausführung das Koppelelement einen magnetisch leitenden Bereich zum Ankoppeln des Gegenelements aufweisen, welcher durch einen Permanentmagneten, wenigstens im Wesentlichen, nur magnetisch beaufschlagt wird, wenn dieser in der Verriegelungsposition ist, bzw. welcher von dem Permanentmagneten magnetisch getrennt ist, wenn dieser in der Entriegelungsposition ist.

Allgemein kann in einer Ausführung das Koppelelement einen magnetisch leitenden Bereich zum Ankoppeln des Gegenelements aufweisen, welcher, insbesondere wahlweise, vorzugsweise durch Bestromen bzw. Nichtbestromen wenigstens eines Elektromagneten und/oder Verstellen wenigstens eines Permanentmagneten in die Verriegelungsposition, durch die Magnetanordnung magnetisch beaufschlagbar ist bzw. beaufschlagt wird. Unter einem magnetisch leitenden Bereich wird vorliegend insbesondere ein Bereich aus einem Material verstanden, das eine Permeabilitätszahl bzw. relative Permeabilität µᵣ aufweist, die wenigstens 10 beträgt, insbesondere einen Bereich aus einem ferromagnetischen Material. Indem ein Permanentmagnet außer magnetischen Eingriff mit dem magnetisch leitenden Bereich des Koppelelements verstellt wird, wird die von dem magnetisch leitenden Bereich auf ein Gegenelement ausgeübte Magnetkraft geschwächt, insbesondere - wenigstens im Wesentlichen - eliminiert, so dass die magnetische Kopplung entfällt. Dadurch kann vorzugsweise ein Verstellweg zum Ab- bzw. Entkoppeln durch Verstellen eines Permanentmagneten in eine Entriegelungsposition reduziert werden. Insbesondere, indem in einer Ausführung ein Koppelelement ein Joch aus einem magnetisch leitenden Material aufweist, um das eine wahlweise bestrombare Spule angeordnet ist, kann in einer Ausführung allgemein die Magnetanordnung, insbesondere ein Elektromagnet, integral mit dem Koppelelement ausgebildet sein.

Zum Koppeln eines Instrumentenmoduls und Instrumententeils eines chirurgischen Instruments wird somit nach einem Aspekt der vorliegenden Erfindung wenigstens ein Elektromagnet der Magnetanordnung des Instrumentenmoduls aktiviert bzw. bestromt und so vorzugsweise eine stromlos geöffnete Ankopplung zwischen Koppel- und Gegenelement geschlossen. Zum Abkoppeln wird der Elektromagnet entsprechend deaktiviert bzw. nicht bestromt.

Zusätzlich oder alternativ kann wenigstens ein Permanentmagnet der Magnetanordnung des Instrumentenmoduls in die Verriegelungsposition verstellt werden. Zum Abkoppeln wird der Permanentmagnet entsprechend in die Entriegelungsposition verstellt.

Insbesondere, um eine stromlos geschlossene Ankopplung zwischen Koppel- und Gegenelement zu schließen, wird in einer Ausführung zum Koppeln eines Instrumentenmoduls und Instrumententeils eines chirurgischen Instruments wenigstens ein Elektromagnet der Magnetanordnung des Instrumentenmoduls, die zusätzlich wenigstens einen Permanentmagneten aufweist, deaktiviert bzw. nicht bestromt. Zum Abkoppeln wird der Elektromagnet entsprechend aktiviert bzw. bestromt.

In einer Ausführung sind Koppel- und Gegenelement bzw. Koppel- und Gegenelementanordnung zusätzlich zu der magnetischen Ankopplung formschlüssig verbindbar bzw. verbunden, vorzugsweise, um sie relativ zueinander zu zentrieren und/oder drehfestzulegen. Insbesondere kann eines von dem Koppel- und dem Gegenelement wenigstens einen exzentrischen Vorsprung aufweisen, der in eine entsprechende Aussparung in dem anderen von dem Koppel- und dem Gegenelement oder einer zwischen diesen angeordneten sterilen Barriere, insbesondere einem Koppelstück einer solchen Barriere, eingreift, wenn Koppel- und Gegenelement, gegebenenfalls über eine Barriere, aneinander gekoppelt sind. Die magnetische Kopplung kann diesen Formschluss in einer Ausführung axial sichern.

Gleichermaßen kann eines von dem Koppel- und dem Gegenelement bolzenartig in einen Hülsen- bzw. Buchsenbereich des anderen von dem Koppel- und dem Gegenelement eingreifen, wenn Koppel- und Gegenelement aneinander gekoppelt sind, insbesondere das Koppelelement bolzenartig in einen Hülsen- bzw. Buchsenbereich des Gegenelements oder das Gegenelement bolzenartig in einen Hülsen- bzw. Buchsenbereich des Koppelelements. Hierdurch können in einer Ausführung Koppel- und Gegenelement senkrecht zu ihrer Längserstreckung formschlüssig festgelegt sein, wobei die magnetische Kopplung sie in Richtung ihrer Längserstreckung kraftschlüssig festlegt.

In einer Ausführung sind Instrumentenmodul und/oder-teil sterilisierbar bzw. steril. Insbesondere, wenn Instrumentenmodul oder -teil einen elektromotorischen Antrieb zum Aktuieren eines Endeffektors des Instruments aufweisen, kann es schwierig sein, dieses zu sterilisieren. Insbesondere daher kann in einer Ausführung zwischen der Koppelelementanordnung und der Gegenelementanordnung eine sterile Barriere angeordnet werden bzw. sein. Die sterile Barriere kann insbesondere, wenigstens im Koppelbereich, flexibel ausgebildet sein, um eine Bewegung von Koppel- und Gegenelementen zur Aktuieren des Endeffektors unter elastischer Deformation mitzumachen.

In einer Ausführung weist die sterile Barriere ein Koppelstück zum magnetischen Ankoppeln eines Gegenelements an ein Koppelelement auf. Das Koppelstück kann mittels einer Dichtung beweglich mit der restlichen Barriere, insbesondere einer Folie, verbunden oder fest mit dieser verbunden, insbesondere integral ausgebildet, sein. Hierdurch kann eine mechanische Kraftübertragung über die Barriere hinweg verbessert werden. In einer Ausführung weist das Koppelstück ein magnetisch leitendes Material auf, um die magnetische Ankopplung zu verbessern.

Insbesondere, wenn Koppel- und Gegenelement, insbesondere formschlüssig, aneinander zentriert sind, kann es vorteilhaft sein, wenn das Koppel- oder Gegenelement mit Spiel in einer Führung des Instrumentenmoduls bzw. -teils gelagert ist. Auf diese Weise kann das Koppel- bzw. Gegenelement beim Ankoppeln einen gewissen Lateralversatz kompensieren.

In einer Ausführung sind Koppel- und Gegenelement stößel- bzw. wellenartig ausgebildet und auf Stoß bzw. stirnseitig aneinander gekoppelt, wobei die Magnetanordnung Koppel- und Gegenelement in Richtung ihrer, vorzugsweise fluchtenden, Längserstreckung gegeneinander zieht, um Zugkräfte bzw. Drehmomente zu übertragen.

In einer Ausführung der vorliegenden Erfindung können Koppel- und Gegenelementanordnung vorteilhafterweise steril, kompakt und/oder - wenigstens im Wesentlichen - spiel- bzw. schlupffrei und/oder ohne visuelle Kontrolle aneinander ge- bzw. voneinander abgekoppelt werden.

Insbesondere, wenn ein Koppelelement und ein Gegenelement magnetisch miteinander drehfest gekoppelt werden, kann dies - insbesondere ohne zusätzliche formschlüssige Verbindung oder bei mehrdeutigen formschlüssigen Verbindungen wie beispielsweise einer Hirth-Verzahnung - dazu führen, dass die Winkelstellung des Gegenelements relativ zum Instrumentenmodul nach dem Ankoppeln nicht eindeutig bekannt ist. Dies ist jedoch insbesondere bei minimalinvasiver Roboterchirurgie, bei der der intrakorporale Endeffektor durch den extrakorporalen Antrieb exakt aktuiert werden soll, erforderlich.

Daher weist nach einem Aspekt der vorliegenden Erfindung, der vorzugsweise mit den vorstehend erläuterten Aspekten kombiniert sein kann, ein Instrumentenmodul eine Koppelelementanordnung mit einem oder mehreren drehbar gelagerten Koppelelementen auf, die lösbar an drehbar gelagerte Gegenelemente einer Gegenelementanordnung eines Instrumententeils angekoppelt werden können, durch die ein Endeffektor eines chirurgischen Instruments aktuierbar ist, welches das Instrumentenmodul und das daran gekoppelte Instrumententeil aufweist.

Koppel- und Gegenelement(anordnung) können in einer Ausführung formschlüssig aneinander gekoppelt bzw. koppelbar sein, insbesondere durch eine Verzahnung, vorzugsweise eine Hirth- oder Stirnradverzahnung. Ist zwischen Koppel- und Gegenelementanordnung eine sterile Barriere angeordnet, können Koppel- und Gegenelement(anordnung) in einer Ausführung formschlüssig mit einem Koppelstück gekoppelt bzw. koppelbar sein, welches vorzugsweise drehbeweglich in der Barriere gelagert ist.

Allgemein sind Koppel- und Gegenelement(anordnung) nach diesem Aspekt der vorliegenden Erfindung in zwei oder mehr unterschiedlichen Orientierungen aneinander koppelbar, wobei als Orientierung vorliegend insbesondere eine Drehlage bzw. -stellung zwischen Koppel- und Gegenelement um deren Drehachse bezeichnet wird. Diese Orientierungen können diskrete Orientierungen sein. In einem einfachen Fall weist eines von einem Koppel- und einem Gegenelement einen oder mehrere Vorsprünge auf, die in wenigstens zwei verschiedenen Orientierungen zwischen Koppel- und Gegenelement in entsprechende Aussparungen in dem anderen von dem Koppel- und dem Gegenelement eingreifen können. Gleichermaßen können Koppel- und Gegenelement korrespondierende Verzahnungen aufweisen, die in um die Zahnteilung versetzten Orientierung ineinander eingreifen und so Koppel- und Gegenelement koppeln können. Die unterschiedlichen Orientierungen können gleichermaßen geometrisch unbestimmt bzw. frei sein, beispielsweise, indem ein Elektromagnet in einem Koppelelement aktiviert wird und einen ferromagnetischen Bereich eines Gegenelements, dessen ebene Stirnseite die ebene Stirnseite des Koppelelements in einer beliebigen Orientierung kontaktiert, in dieser Orientierung fixiert.

Insbesondere, um bei solchen Ankopplungen, die in mehreren Orientierungen zwischen Koppel- und Gegenelement möglich sind, den Endeffektor durch einen Antrieb besser, vorzugsweise ohne vorherige Rekalibrierung, aktuieren zu können, weist das Instrumentenmodul in einer Ausführung einen Winkelsensor zum Erfassen einer Winkelstellung der angekoppelten Gegenelementanordnung, insbesondere eines Senders, an, insbesondere in, einem oder mehreren Gegenelementen, auf. Der Winkelsensor kann dabei mehrere Einzelsensoren zum Erfassen je einer Winkelstellung eines angekoppelten Gegenelements aufweisen. Das bzw. die Gegenelemente der Gegenelementanordnung weisen entsprechend jeweils drehfeste Sender auf, die dazu eingerichtet sind, durch den Winkelsensor des Instrumentenmoduls, insbesondere einen Einzelsensor, erfasst zu werden.

Zur kompakteren Darstellung wird vorliegend auch die Menge der Winkelstellungen von zwei oder mehr, insbesondere allen Gegenelementen der Gegenelementanordnung verallgemeinernd als eine Winkelstellung der Gegenelementanordnung im Sinne der vorliegenden Erfindung bezeichnet. Eine Winkelstellung eines Gegenelements kann insbesondere eine Orientierung bzw. Drehstellung dieses Gegenelements relativ zu dem Instrumentenmodul, insbesondere relativ zu einer gehäuse- oder drehlagerfesten Referenz sein. Gleichermaßen kann eine Winkelstellung eines Gegenelements eine Orientierung bzw. Drehstellung dieses Gegenelements relativ zu dem angekoppelten Koppelelement sein.

In einer Ausführung ist der Winkelsensor dazu eingerichtet, zusätzlich eine Orientierung bzw. Drehstellung des an das Gegenelement angekoppelten Koppelelements relativ zu dem Instrumentenmodul, insbesondere relativ zu einer gehäuse- oder drehlagerfesten Referenz zu erfassen. In einer Weiterbildungkann daraus, insbesondere durch vorzeichenrichtige Addition, mit der Winkelstellung des Gegenelements relativ zu dem angekoppelten Koppelelement die Winkelstellung des Gegenelements relativ zu einer gehäuse- oder drehlagerfesten Referenz ermittelt werden. Gleichermaßen kann umgekehrt mit der Winkelstellung des Gegenelements relativ zu einer gehäuse- oder drehlagerfesten Referenz die Winkelstellung des Gegenelements relativ zu dem angekoppelten Koppelelement erfasst bzw. ermittelt werden.

Der Winkelsensor kann zum berührungslosen Erfassen der Winkelstellung der Gegenelementanordnung bzw. der Winkelstellung(en) des Senders bzw. der Sender eingerichtet sein. Er kann insbesondere ein magnetischer, elektrischer, kapazitiver und/oder optischer Winkelsensor sein. Der bzw. die Sender können aktiv oder passiv sein und insbesondere einen Permanentmagneten, dessen Nord-Süd-Achse vorzugsweise, wenigstens im Wesentlichen, senkrecht zur Drehachse des Gegenelements orientiert sein kann, einen Transponder, vorzugsweise eines RFID-Systems, eine optische Markierung oder dergleichen aufweisen.

In einer Ausführung ist der Winkelsensor als Absolutwertgeber bzw. dazu ausgebildet, eine absolute Winkelstellung der Gegenelementanordnung relativ zu Instrumentenmodul bzw. Koppelelement zu erfassen, beispielsweise mittels eines absolutcodierten Senders oder Empfängers. Die Kodierung kann einen Winkelbereich von 360° aufweisen (sogenannte Singleturn- Absolutwertgeber), so dass der Winkelsensor zwei um 360° verdrehte Winkelstellungen als dieselbe Winkelstellung erfasst. In einer anderen Ausführung kann die Kodierung einen Winkelbereich von mehr als 360° aufweisen (sogenannte Multiturn- Absolutwertgeber), so dass der Winkelsensor zwei um 360° verdrehte Winkelstellungen als unterschiedliche Winkelstellungen erfasst. Vorzugsweise steht die Winkelstellung bei einem Absolutwertgeber unmittelbar nach dem Ankoppeln und Auslesen des Winkelsensors zur Verfügung, ohne dass eine Verdrehung der Gegenelementanordnung erforderlich ist.

In einer anderen Ausführung ist der Winkelsensor als Inkrementalwertgeber bzw. dazu ausgebildet, nur eine Winkeländerung der Gegenelementanordnung relativ zu Instrumentenmodul bzw. Koppelelement zu erfassen. In einer Weiterbildung ist der Sender oder Empfänger abstandscodiert bzw. weist eine oder mehrere Referenzmarken auf. Nach Überfahren einer Referenzmarke kann die Winkelstellung dann durch Integration bzw. Aufsummierung der Inkrementalwerte bzw. Winkeländerung erfasst werden.

In einer Ausführung weist ein chirurgisches Instrument mehrere Instrumententeile auf, die wahlweise an das Instrumentenmodul angekoppelt werden können, wobei die Gegenelementanordnungen der verschiedenen Instrumententeile unterschiedlich codierte Sender aufweisen, die dazu eingerichtet sind, durch den Winkelsensor des Instrumentenmoduls erfasst zu werden, wobei der Winkelsensor zusätzlich dazu eingerichtet ist, die Codierung der Sender zu erfassen und so das angekoppelte Instrumententeil zu identifizieren. Dadurch können die Funktionalitäten, einerseits die Orientierung der Gegenelementanordnung zu bestimmen, und andererseits das angekoppelte Instrumententeil zu identifzieren, durch den- bzw. dieselben Sender und Winkelsensoren implementiert werden. Ein Permanentmagnet kann in einer Ausführung gleichermaßen als Sender zum Erfassen der Winkelstellung und zum magnetischen Ankoppeln dienen bzw. verwendet werden.

Beim oder nach dem Ankoppeln des Instrumententeils an das Instrumentenmodul in einer von mehreren Orientierungen wird nach einem Aspekt der vorliegenden Erfindung die Winkelstellung der angekoppelten Gegenelementanordnung des Instrumententeils, insbesondere relativ zur Koppelelementanordnung oder einer instrumentenmodulgehäusefesten Referenz, durch den Winkelsensor des Instrumentenmoduls erfasst. Auf diese Weise ist nach dem Erfassen die Orientierung des bzw. der Gegenelemente und damit vorzugsweise auch eine Stellung bzw. Koordinate des Endeffektors bekannt, so dass der Endeffektor in einer Ausführung durch einen Antrieb ohne Rekalibrierung korrekt aktuiert werden kann.

In einer Ausführung wird ein Zusammenhang, insbesondere ein Kalibrier-Offset, zwischen der Winkelstellung der Gegenelementanordnung und einer Endeffektorstellung vorab erfasst und abgespeichert. Nach Ankoppeln der Gegenelementanordnung kann die Endeffektorstellung aus der durch den Winkelsensor erfassten Winkelstellung der Gegenelementanordnung unter Berücksichtigung dieses Zusammenhangs ermittelt werden.

Die vorliegende Erfindung betrifft insbesondere folgende Ausgestaltungen:
Eine erste Ausgestaltung betrifft ein Chirurgierobotersystem mit einem Roboter; und einer, insbesondere lösbar, an dem Roboter befestigten Instrumentenanordnung nach einer der Ausgestaltungen zwei bis fünf.

Ein zweite Ausgestaltung betrifft eine Instrumentenanordnung zur, insbesondere lösbaren, Befestigung an dem Roboter eines Chirurgierobotersystems nach Ausgestaltung 1, mit einer Antriebseinheit nach einer der Ausgestaltungen sechs bis neun, einem Instrument nach einer der Ausgestaltungen zehn bis vierzehn, das mit der Antriebseinheit lösbar verbunden ist; und einer Instrumenten-Schnittstelle nach einer der Ausgestaltungen fünfzehn bis siebzehn, die zwischen Antriebseinheit und Instrument angeordnet ist.

Eine dritte Ausgestaltung betrifft eine Instrumentenanordnung nach Ausgestaltung zwei, mit einem Zusatz-Instrument und/oder einem Antriebsmittel zum Betätigen eines Endeffektors, welches, insbesondere lösbar, durch das Instrument der Instrumentenanordnung eingeführt ist.

Eine vierte Ausgestaltung betrifft eine Instrumentenanordnung nach einer der Ausgestaltungen zwei bis drei, wobei wenigstens eine Antriebswelle der Antriebseinheit und eine Antriebswelle des Instruments koaxial, insbesondere fluchten angeordnet sind.

Eine fünfte Ausgestaltung betrifft eine Instrumentenanordnung nach einer der Ausgestaltungen zwei bis vier, wobei ein Koppelteil einer Antriebswelle der Antriebseinheit und ein Koppelteil einer Antriebswelle des Instruments magnetisch miteinander gekoppelt sind.

Eine sechste Ausgestaltung betrifft eine Antriebseinheit für eine Instrumentenanordnung nach einer der Ausgestaltungen zwei bis fünf, mit wenigstens einem Drehantrieb mit einer Antriebswelle, insbesondere Antriebs-Hohlwelle, mit einem Koppelteil zur Koppelung mit einer Antriebswelle des Instruments.

Eine siebte Ausgestaltung betrifft eine Antriebseinheit nach Ausgestaltung sechs, mit wenigstens einer inneren Antriebswelle, insbesondere Antriebs-Hohlwelle, die konzentrisch in einer äußeren Antriebs-Hohlwelle angeordnet, insbesondere gelagert ist

Eine achte Ausgestaltung betrifft eine Antriebseinheit nach einer der Ausgestaltungen sechs bis sieben, wobei wenigstens ein Drehantrieb koaxial, insbesondere fluchtend oder parallel versetzt, oder winkelig, insbesondere rechtwinkelig, zu seiner Antriebswelle angeordnet ist.

Eine neunte Ausgestaltung betrifft eine Antriebseinheit nach einer der Ausgestaltungen sechs bis acht, wobei das Koppelteil axial verschieblich, insbesondere vorgespannt, an der Antriebswelle angeordnet ist.

Eine zehnte Ausgestaltung betrifft ein Instrument für eine Instrumentenanordnung nach einer der Ausgestaltungen zwei bis fünf, mit einem Instrumentenschaft und wenigstens einer Antriebswelle, insbesondere Antriebs-Hohlwelle, mit einem Koppelteil zur Koppelung mit einer Antriebswelle der Antriebseinheit.

Eine elfte Ausgestaltung betrifft ein Instrument nach Ausgestaltung zehn, mit wenigstens einer inneren Antriebswelle, insbesondere Antriebs-Hohlwelle, die konzentrisch in einer äußeren Antriebs-Hohlwelle angeordnet, insbesondere gelagert ist.

Eine zwölfte Ausgestaltung betrifft ein Instrument nach einer der Ausgestaltungen zehn bis elf, wobei die Antriebswelle koaxial, insbesondere fluchtend oder parallel versetzt, oder winkelig, insbesondere rechtwinkelig, zu dem Instrumentenschaft angeordnet ist.

Eine dreizehnte Ausgestaltung betrifft ein Instrument nach einer der Ausgestaltungen zehn bis zwölf, mit einem Umsetzungsgetriebe zur Umsetzung einer Rotation einer Antriebswelle in eine Translation eines Zug- und/oder Schubmittels.

Eine vierzehnte Ausgestaltung betrifft ein Instrument nach Ausgestaltung dreizehn, wobei das Umsetzungsgetriebe eine Kulissenführung aufweist und/oder in einer antriebseinheitszu- oder -abgewandten Hälfte des Instrumentenschaftes angeordnet ist.

Eine fünfzehnte Ausgestaltung betrifft eine Instrumenten-Schnittstelle für eine Instrumentenanordnung nach einer der Ausgestaltung zwei bis fünf, mit einer Hülle, insbesondere nach Ausgestaltung achtzehn, zum Umhüllen der Antriebseinheit der Instrumentenanordnung.

Eine sechzehnte Ausgestaltung betrifft eine Instrumenten-Schnittstelle nach Ausgestaltung fünfzehn, wobei die Hülle eine Innendurchführung für ein durch das Instrument und die Antriebseinheit der Instrumentenanordnung eingeführtes Zusatz-Instrument aufweist.

Eine siebzehnte Ausgestaltung betrifft eine Instrumenten-Schnittstelle nach einer der Ausgestaltungen fünfzehn bis sechzehn, mit wenigstens einem drehbaren, insbesondere kegelförmigen, Zwischenelement zur reib- oder formschlüssigen Koppelung mit dem Koppelteil einer Antriebswelle der Antriebseinheit und/oder des Instruments.

Eine achtzehnte Ausgestaltung betrifft eine Hülle mit einer schlauchartigen Innendurchführung zum Durchführen durch eine Hohlwelle eines Roboters oder einer Antriebseinheit nach einer der Ausgestaltungen sechs bis neun und durch eine Austrittsöffnung der Hülle, wobei die Innendurchführung einen Blindstopfen und einen Abschlussring zur Befestigung an einem Umfangsbereich der Innendurchführung aufweist, der durch Entfernen des Blindstopfens frei wird.

Eine neunzehnte Ausgestaltung betrifft ein Verfahren zum Umhüllen eines Roboters oder einer Antriebseinheit nach einer der Ausgestaltungen sechs bis neun mittels einer Hülle nach der vorhergehenden Ausgestaltung, wobei die mit dem Blindstopfen versehene Innendurchführung durch eine Hohlwelle und die Austrittsöffnung der Hülle durchgeführt, anschließend entfernt und der Abschlussring an dem Umfangsbereich der Innendurchführung befestigt wird, der durch das Entfernen des Blindstopfens frei geworden ist.

Eine zwanzigste Ausgestaltung betrifft ein Instrumentenmodul mit einer Koppelelementanordnung mit wenigstens einem Koppelelement zum lösbaren Ankoppeln eines Gegenelements einer Gegenelementanordnung zum Aktuieren eines Endeffektors eines chirurgischen Instruments, wobei das Koppelelement eine Magnetanordnung zum magnetischen Ankoppeln des Gegenelements aufweist.

Eine einundzwanzigste Ausgestaltung betrifft ein Instrumentenmodul nach der vorhergehenden Ausgestaltung, wobei das Koppelelement zum Aktuieren des Endeffektors translatorisch und/oder rotatorisch beweglich ist.

Eine zweiundzwanzigste Ausgestaltung betrifft ein Instrumentenmodul nach einer der vorhergehenden Ausgestaltungen, wobei die Magnetanordnung wenigstens einen wahlweise bestrombaren Elektromagneten aufweist.

Eine dreiundzwanzigste Ausgestaltung betrifft ein Instrumentenmodul nach einer der vorhergehenden Ausgestaltungen, wobei die Magnetanordnung wenigstens einen Permanentmagneten aufweist.

Eine vierundzwanzigste Ausgestaltung betrifft ein Instrumentenmodul nach der vorhergehenden Ausgestaltung, wobei der Permanentmagnet an dem Koppelelement, insbesondere elektromotorisch, hydraulisch, pneumatisch und/oder manuell, zwischen einer Verriegelungs- und einer hiervon verschiedenen Entriegelungsposition verstellbar ist.

Eine fünfundzwanzigste Ausgestaltung betrifft ein Instrumentenmodul nach einer der vorhergehenden Ausgestaltungen, wobei das Koppelelement einen magnetisch leitenden Bereich zum Ankoppeln des Gegenelements aufweist, welcher, insbesondere wahlweise, durch die Magnetanordnung magnetisch beaufschlagt wird.

Eine sechsundzwanzigste Ausgestaltung betrifft ein Instrumentenmodul nach der vorhergehenden Ausgestaltung, wobei es einen Antrieb zum Aktuieren der Koppelelementanordnung oder einen in einen Patienten einführbaren Instrumentenschaft mit einem Endeffektor aufweist, der durch die Koppelelementanordnung aktuierbar ist.

Eine siebenundzwanzigste Ausgestaltung betrifft ein Instrumentenmodul, insbesondere nach einer der vorhergehenden Ausgestaltungen, mit einer Koppelelementanordnung mit wenigstens einem Koppelelement zum lösbaren Ankoppeln eines Gegenelements einer Gegenelementanordnung zum Aktuieren eines Endeffektors eines chirurgischen Instruments, wobei die Koppel- und Gegenelementanordnung in wenigstens zwei unterschiedlichen Orientierungen koppelbar sind, mit einem Winkelsensor zum, insbesondere berührungslosen, Erfassen einer Winkelstellung der angekoppelten Gegenelementanordnung.

Eine achtundzwanzigste Ausgestaltung betrifft ein Instrumententeil mit einer Gegenelementanordnung mit wenigstens einem Gegenelement zum Ankoppeln eines Koppelelements der Koppelelementanordnung eines Instrumentenmoduls nach einer der vorhergehenden Ausgestaltungen, wobei das Gegenelement einen magnetisch beaufschlagbaren Bereich zum magnetischen Ankoppeln des Koppelelements und/oder einen drehfesten Sender zum Erfassen durch den Winkelsensor des Instrumentenmoduls aufweist.

Eine neunundzwanzigste Ausgestaltung betrifft ein chirurgisches Instrument mit einem Instrumentenmodul nach einer der vorhergehenden Ausgestaltungen und einem damit lösbar verbindbaren Instrumententeil nach der vorhergehenden Ausgestaltung.

Eine dreißigste Ausgestaltung betrifft ein chirurgisches Instrument nach der vorhergehenden Ausgestaltung, wobei Koppel- und Gegenelementanordnung formschlüssig verbindbar sind.

Eine einunddreißigste Ausgestaltung betrifft ein chirurgisches Instrument nach einer der vorhergehenden Ausgestaltungen, wobei zwischen der Koppelelementanordnung und der Gegenelementanordnung eine sterile Barriere angeordnet ist.

Eine zweiunddreißigste Ausgestaltung betrifft ein chirurgisches Instrument nach der vorhergehenden Ausgestaltung, wobei die sterile Barriere ein, insbesondere magnetisch leitendes, Koppelstück zum, insbesondere magnetischen, Ankoppeln der Gegenelementanordnung an die Koppelelementanordnung aufweist.

Eine dreiunddreißigste Ausgestaltung betrifft ein chirurgisches Instrument nach einer der vorhergehenden Ausgestaltungen, wobei die Koppel- oder Gegenelementanordnung mit Spiel in einer Führung gelagert ist.

Eine vierunddreißigste Ausgestaltung betrifft ein Verfahren zum Koppeln eines Instrumentenmoduls und Instrumententeils eines chirurgischen Instruments nach einer der vorhergehenden Ausgestaltungen, mit den Schritten Aktivieren oder Deaktivieren eines Elektromagneten der Magnetanordnung des Instrumentenmoduls und/oder Verstellen eines Permanentmagneten der Magnetanordnung des Instrumentenmoduls in die Verriegelungsposition und/oder Erfassen einer Winkelstellung der angekoppelten Gegenelementanordnung des Instrumententeils durch den Winkelsensor des Instrumentenmoduls.

Weitere Vorteile und Merkmale ergeben sich aus den Unteransprüchen und den Ausführungsbeispielen. Hierzu zeigt, teilweise schematisiert:
- Fig. 1:: einen Teil einer Instrumentenanordnung eines Chirurgierobotersystems nach einer Ausführung der Erfindung in einem perspektivischen Schnitt;
- Fig. 2:: ein instrumentenseitiges Umsetzungsgetriebe nach einer weiteren Ausführung der vorliegenden Erfindung;
- Fig. 3:: das Umsetzungsgetriebe der Fig. 2 in einem anderen perspektivischen Schnitt;
- Fig. 4:: einen Schnitt einer Kegelkupplung einer Instrumentenanordnung nach einer Ausführung der vorliegenden Erfindung;
- Fig. 5:: weitere Ausführungen einer solchen Kegelkupplung;
- Fig. 6:: eine Wellenkupplung einer Instrumentenanordnung nach einer Ausführung der vorliegenden Erfindung;
- Fig. 7:: einen Schnitt dieser Wellenkupplung der Fig. 6;
- Fig. 8:: eine Magnetkupplung einer Instrumentenanordnung nach einer Ausführung der vorliegenden Erfindung;
- Fig. 9:: die Magnetkupplung der Fig. 8 in einem Schnitt;
- Fig. 10:: in zwei perspektivischen Ansichten ein Umsetzgetriebe nach einer Ausführung der vorliegenden Erfindung;
- Fig. 11:: einen vergrößerten Schnitt des Umsetzgetriebes der Fig. 10;
- Fig. 12:: ein Instrument nach einer Ausführung der vorliegenden Erfindung mit einem distalen Umsetzgetriebe in einer perspektivischen Gesamtansicht (oben) bzw. einem vergrößerten Teilschnitt (unten);
- Fig. 13:: eine Instrumenten-Schnittstelle nach einer Ausführung der vorliegenden Erfindung;
- Fig. 14:: die Instrumenten-Schnittstelle der Fig. 13 mit verbundenem Blindstopfen;
- Fig. 15:: die Instrumenten-Schnittstelle der Fig. 14 mit entferntem Blindstopfen;
- Fig. 16:: die Instrumenten-Schnittstelle der Fig. 15 mit verbundenem Abschlussring;
- Fig. 17:: die Instrumenten-Schnittstelle der Fig. 16 mit ankoppelndem Instrument;
- Fig. 18:: eine Instrumentenanordnung mit einem eingeführten Zusatz-Instrument nach einer Ausführung der vorliegenden Erfindung;
- Fig. 19:: einen Teil eines Instruments einer Instrumentenanordnung nach einer weiteren Ausführung der vorliegenden Erfindung;
- Fig. 20:: einen Teil eines Instruments einer Instrumentenanordnung nach einer weiteren Ausführung der vorliegenden Erfindung;
- Fig. 21A, 21B:: ein Umhüllen eines Roboters mit einer Hülle nach einer Ausführung der vorliegenden Erfindung;
- Fig. 22:: einen Teil eines chirurgischen Instruments nach einer Ausführung der vorliegenden Erfindung in einem Längsschnitt;
- Fig. 23:: einen Teil eines chirurgischen Instruments nach einer weiteren Ausführung der vorliegenden Erfindung in Fig. 1 entsprechender Darstellung;
- Fig. 24:: einen Teil eines chirurgischen Instruments nach einer weiteren Ausführung der vorliegenden Erfindung in Fig. 1, 2 entsprechender Darstellung;
- Fig. 25:: einen Teil eines chirurgischen Instruments nach einer weiteren Ausführung der vorliegenden Erfindung; und
- Fig. 26:: einen Teil eines chirurgischen Instruments nach einer weiteren Ausführung der vorliegenden Erfindung in Fig. 4 entsprechender Darstellung.

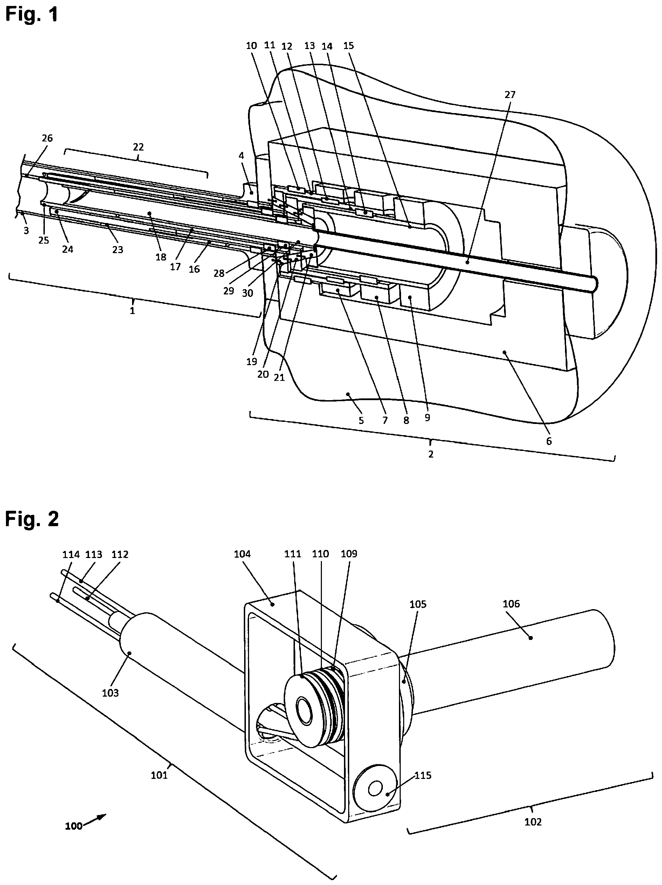

Fig. 1 zeigt einen Teil einer Instrumentenanordnung eines Chirurgierobotersystems nach einer Ausführung der Erfindung in einem perspektivischen Schnitt.

Die Instrumentenanordnung umfasst ein Instrument 1, eine damit verbundene Antriebseinheit 2 und eine zwischen Antriebseinheit und Instrument angeordnete Instrumenten-Schnittstelle mit einer sterilen Hülle 5.

In diesem Ausführungsbeispiel fallen die Drehachsen der Antriebseinheit mit einer Schaftachse des Instruments zusammen. Dieses Konzept eignet sich insbesondere für Instrumente, die mit Zug-/Druckstangen aktuiert werden.

Das sterile chirurgische Instrument 1 ist auf der in Fig. 1 linken, die Antriebseinheit 2 auf der in Fig. 1 rechten Seite dargestellt. Das Instrument 1 ist über einen Anschlussflansch 4 an einem proximalen Ende des Instrumentenschafts 3 mit einem Gehäuse 6 der Antriebseinheit 2 mechanisch lösbar verbunden. Die Antriebseinheit 2 ist von einer sterilen Hülle 5 umgeben, um die Kontamination des Operationsgebiets zu verhindern.

Im Gehäuse 6 der Antriebseinheit 2 befinden sich in diesem Ausführungsbeispiel drei unabhängige Drehantriebe mit jeweils einer Antriebswelle 10,13 bzw. 15 und einem zugehörigen Elektromotor 7, 8 bzw. 9. Die Antriebswellen 10, 13, 15 sind als Hohlwellen ausgeführt und zueinander koaxial angeordnet. Antriebswelle 10 ist an einer Lagerstelle 11 im Gehäuse 6 vollwertig gelagert. Die innere Antriebswelle 13 ist mit einem Lager 12 in der Antriebswelle 10, die Antriebswelle 15 mit einem Lager 14 in der Antriebswelle 13 gelagert. Dieses Konzept ermöglicht vorteilhaft einen vor allem in radialer Richtung sehr kompakten Aufbau der trennbaren Instrument-Schnittstelle. In einer Mehrroboter-Anwendung kann somit das Risiko von Kollisionen zwischen einzelnen Robotern aufgrund des geringeren zulässigen Mindestabstands zwischen den Instrumenten deutlich reduziert werden.

Die symbolischen Darstellungen der Elektromotoren 7, 8, 9 beinhalten weitere für einen geregelten Betrieb erforderliche Komponenten wie beispielsweise Getriebe und/oder Sensoren. Bevorzugte Ausführungen sind konzentrisch angeordnete Motoreinheiten, die entweder als Direktantriebe oder als Motoren mit nachgeschaltetem Untersetzungsgetriebe, zum Beispiel Planetengetrieben oder Harmonic-Drive Getrieben realisiert sein können.

In einer nicht dargestellten Abwandlung können die Drehantriebe radial versetzte Elektromotoren, die die Antriebswellen jeweils mit einem Stirnrad- oder Reibradgetriebe antreiben, oder orthogonal versetzte Elektromotoren aufweisen, die die Antriebswellen jeweils mit einem Schnecken-, Schraubenrad-, oder Kronenradgetriebe antreiben.

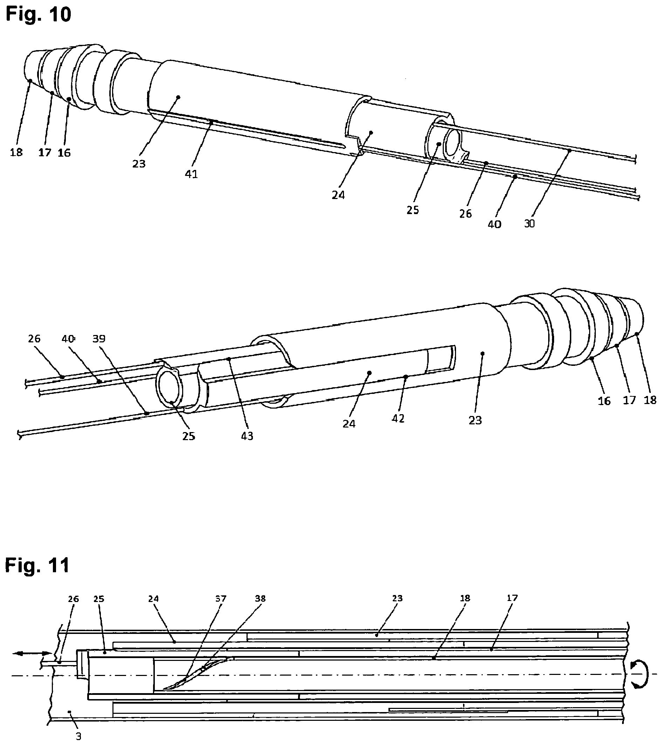

Die ineinander geschachtelten Antriebswellen 10, 13 und 15 werden auf Instrumentenseite in Form der Antriebswellen 16, 17 bzw. 18 fortgeführt, die ebenfalls als Hohlwellen ausgeführt und zueinander koaxial angeordnet sind. Die Lagerung der instrumentenseitigen Antriebswellen 16, 17 und 18 ist als Fest-Los-Lagerung ausgebildet, wobei Festlager 28, 29 und 30 am proximalen Ende des Instrumentenschafts 3 angeordnet sind. Welle 16 ist an der Lagerstelle 28 im Instrumentenschaft 3 radial und axial gelagert. Die innenliegende Antriebswelle 17 ist mit dem Lager 29 in der Antriebswelle 16, die Antriebswelle 18 mit dem Lager 30 in Welle 17 abgestützt. Als Loslager fungieren Schiebehülsen 23, 24 und 25, die zugleich Bestandteile eines instrumentenseitigen Umsetzgetriebes 22 zur Umwandlung der rotatorischen Antriebsbewegung in eine translatorische Bewegung von Zug- und/oder Schubmittel 26, 39 bzw. 40 (vgl. Fig. 10). Diese übertragen schließlich die Antriebsbewegung zu den Instrumenten- bzw. Endeffektorfreiheitsgraden am distalen Ende des Instrumentenschafts 3.

Fig. 1 zeigt exemplarisch nur ein Zug- und/oder Schubmittel 26, wenngleich für jeden Freiheitsgrad des Instruments ein eigenes Übertragungsglied vorgesehen ist. Beispiele für derartige Zug- und/oder Schubmittel sind Seilzüge, Bowdenzüge oder Zug-/Druckstangen.

Zur Verbindung der Antriebswellen 10, 13 und 15 der Antriebseinheit mit den instrumentenseitigen Antriebswellen 16, 17 und 18 ist ein Kupplungsmechanismus vorgesehen, der zugleich eine Sterilbarriere zwischen dem Instrument und der unsterilen Antriebseinheit darstellt. Die in Fig. 1 exemplarisch dargestellte Kupplung ist eine Kegel-Kupplung, die die Antriebsmomente durch Reib- oder Formschluss überträgt.

Durch dieses Konstruktionsprinzip können auch die in der koaxialen Anordnung innenliegenden Antriebswellen 15 und 18 als Hohlwellen ausgeführt werden. Somit verbleibt im Zentrum des Instrumentenschafts 3 ausreichend Platz, um weitere Antriebsmittel, beispielsweise einen Bowdenzug, eine Drehwelle mit biegsamem Abschnitt im Bereich eines Mehrfachgelenks zum Antrieb eines Endeffektors und/oder ein Zusatz-Instrument, insbesondere eine elektrische Leitung, einen Schlauch oder dergleichen durchzuführen. Eine weitere mögliche Anwendung dieses Konstruktionsprinzips ist das Einführen von chirurgischen Spezial-Instrumenten durch das Zentrum des Instrumentenschafts.

Um die Sterilität der durch die Instrumentenmitte durchgeführten Elemente auch im Bereich der Antriebseinheit 2 zu gewährleisten, erstreckt sich die Sterilbarriere mit einer Innendurchführung in Form eines sterilen Führungsrohrs 27 durch die gesamte Antriebseinheit 2, wie nachfolgend beschrieben.

Fig. 2 zeigt ein instrumentenseitiges Umsetzungsgetriebe 100 nach einer weiteren Ausführung der vorliegenden Erfindung, bei der Antriebswellen- und Schaftachse orthogonal sind. Diese Anordnung eignet sich besonders für Instrumente, die mit Seilzügen aktuiert werden. Sie kann jedoch auch für Instrumente mit Zug-/Druckstangen eingesetzt werden, indem die instrumentenseitigen Getriebe zur Umsetzung der Rotation einer Antriebswelle in eine Translation eines Zug- und/oder Schubmittels beispielsweise als Schubkurbelmechanismus realisiert sein kann.

Am proximalen Ende des Instruments 101 befindet sich ein Gehäuse 104, das mit dem Instrumentenschaft 103 unlösbar verbunden ist. Das Instrument 101 ist am proximalen Ende mit der Antriebseinheit 102 (dessen Gehäuse nicht dargestellt ist) über eine Sterilbarriere 105 verbunden. Die Antriebswellen 106, 107 und 108 sind in der Antriebseinheit 102 koaxial angeordnet, um möglichst kompakte Abmessungen zu erreichen. Sie werden auf Instrumentenseite jeweils als Seilrolle 109, 110 bzw. 111 fortgeführt. Die Verbindung der Wellenstücke geschieht jeweils mit den sterilen Kupplungs-Zwischensegmenten 116, 117 und 118, die zueinander drehbar sind.

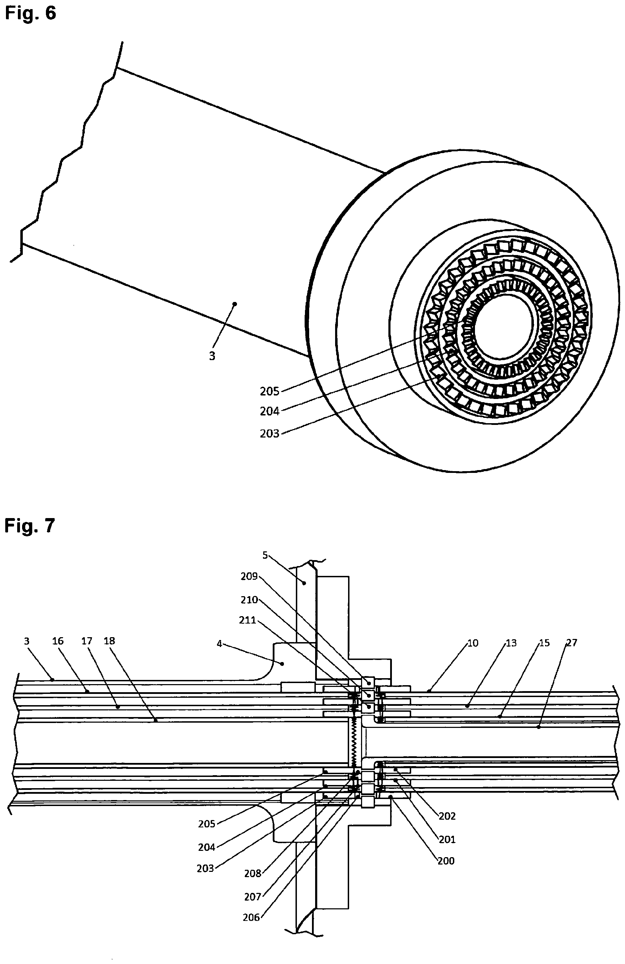

Ein Kupplungs-Zwischenelement 118 der außenliegenden Antriebswelle 106 ist mit der Sterilbarriere 105 verbunden und in dieser drehbar gelagert. Im Ausführungsbeispiel sind die Antriebswellen von Antriebseinheit und Instrument mittels einer Stirnzahnkupplung formschlüssig gekoppelt, die mit Bezug auf Fig. 6 genauer beschrieben wird.

Die Seilzüge 112, 113 und 114, die die Instrument-Freiheitsgrade aktuieren, sind um die instrumentenseitigen Seilrollen bzw. Antriebswellen 109, 110 bzw. 111 gewickelt, sodass der Kraftfluss zwischen den Antriebswellen 106, 107 und 108 und den Instrument-Freiheitsgraden geschlossen ist. Optional kann eine Rohrdurchführung 115 vorgesehen sein, die beispielsweise zur Führung eines Zusatz-Instruments, insbesondere von Medienleitungen, zum distalen Ende des Instrumentenschafts 103 verwendet werden kann.

### Lösbare Kupplung mit Sterilbarriere für mindestens einen rotatorischen Antriebsstrang

Zur Anbindung des Instruments an die Antriebseinheit ist ein einfach lösbarer Kupplungsmechanismus vorgesehen, der zugleich die Sterilbarriere zwischen dem Instrument und der unsterilen Antriebseinheit darstellt.

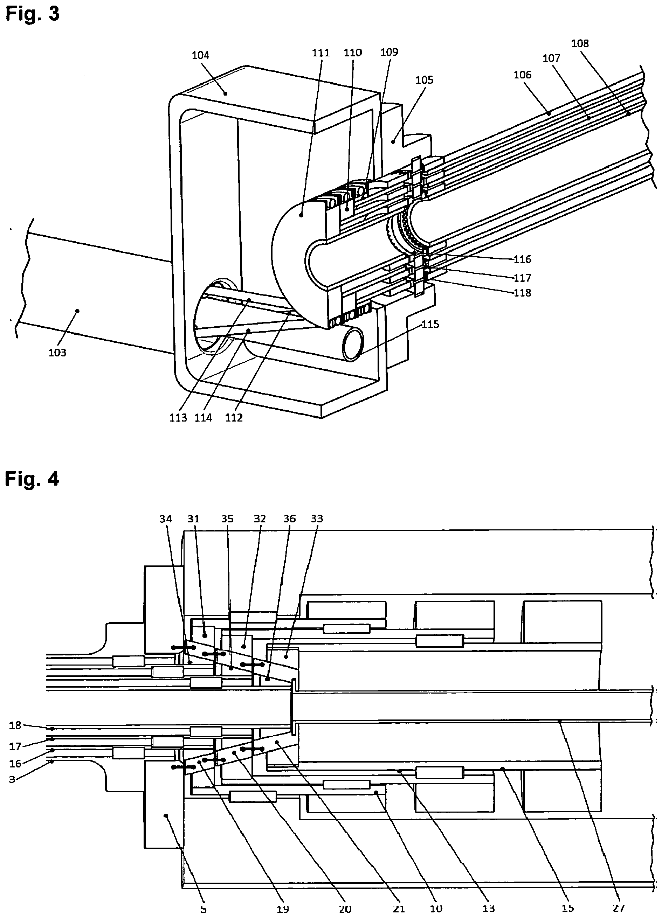

Fig. 4 zeigt in einem Schnitt die Kegelkupplung des Ausführungsbeispiels der Fig. 1 mit Sterilbarriere. Die Kupplungsanordnung überträgt die Antriebsmomente von den Antriebswellen 10, 13 und 15 durch Reib- oder Formschluss auf die instrumentenseitigen Antriebswellen 16, 17 und 18. An den proximalen Enden der instrumentenseitigen Hohlwellen 16, 17 und 18 sind Koppelteile in Form von Aussenkegel 34, 35 und 36 angeordnet, die mit der jeweiligen Hohlwelle fest verbunden sind. An den distalen Enden der Antriebswellen 10, 13 und 15 sind Koppelteile 31, 32, 33 mit Innenkegeln angeordnet. Die Verbindung der Wellenenden erfolgt über kegelförmige Zwischenelemente 19, 20 bzw. 21, die als Sterilbarriere fungieren. Diese Elemente sind miteinander und auch mit der Sterilbarriere 5 abgedichtet verbunden. Diese Verbindungen dienen lediglich der einfachen Handhabung bei der Installation der Sterilbarriere, lassen aber ansonsten alle zur Funktion erforderlichen Bewegungen der Zwischenelemente zu, insbesondere eine Drehung mit den Antriebswellen. Zugleich stellen diese Zwischenelemente 19, 20 bzw. 21 eine Spalt- bzw. Labyrinthdichtung zwischen den Koppelteilen dar.

Die an den Antriebswellen 10, 13 und 15 angeordneten Koppelteile 31, 32, 33 sind jeweils mit einer Welle drehfest, jedoch axial verschieblich verbunden, zum Beispiel durch ein Zahn- oder Polygonwellenprofil. Somit kann die zur Kraftübertragung nötige axiale Vorspannkraft beispielsweise durch Federn, die auf die antriebsseitigen Koppelteile wirken, aufgebracht werden. Zugleich wird ein möglicher Axialversatz zwischen den antriebs- und instrumentenseitigen Wellenabschnitten ausgeglichen.

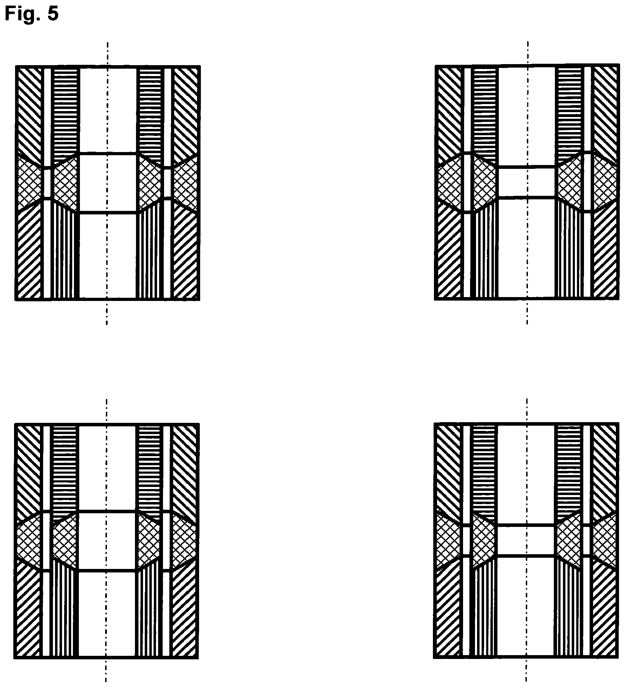

Anstelle der Kombination von antriebsseitigen Innenkegeln und instrumentenseitigen Aussenkegeln sind für jede Paarung einer inneren und einer äußeren Antriebswelle von Antriebseinheit und Instrument noch weitere Anordnungen denkbar, die in Fig. 5 skizziert sind:

| Koppelteil 31/32/33, 34/35/36 | Fig. 5 oben links | Fig. 5 oben rechts | Fig. 5 unten links | Fig. 5 unten rechts |
|---|---|---|---|---|
| innere Antriebshohlwelle der Antriebseinheit | Innenkegel | Außenkegel | Innenkegel | Außenkegel |
| innere Antriebshoh lwelle des Instruments | Innenkegel | Außenkegel | Innenkegel | Außenkegel |
| äußere Antriebshohlwelle der Antriebseinheit | Außenkegel | Innenkegel | Innenkegel | Außenkegel |
| äußere Antriebshohlwelle des Instruments | Außen kegel | Innenkegel | Innenkegel | Außenkegel |

Fig. 6 zeigt eine Wellenkupplung mit Sterilbarriere, die die Antriebsmomente formschlüssig über Stirnverzahnungen zum Beispiel eine Hirth-Verzahnung von den Antriebswellen 10, 13 und 15 auf die instrumentenseitigen Antriebswellen 16, 17 und 18 überträgt, Fig. 7 einen Schnitt dieser Wellenkupplung. Die Wellenkupplung kann anstelle der Kegelkupplung der Fig. 4, 5 insbesondere bei einer Instrumentenanordnung nach Fig. 1 oder 2, 3 vorgesehen sein.

Hierzu sind an den proximalen Koppelteilen der instrumentenseitigen Hohlwellen 16, 17 und 18 Stirnverzahnungen 203, 204, 205 aufgebracht, die mit der jeweiligen Hohlwelle fest verbunden sind. An den distalen Enden der Antriebswellen 10, 13, 15 sind Koppelteile in Form von Schiebehülsen mit Stirnverzahnung 200, 201, 202 angeordnet. Die Verbindung der Wellenenden erfolgt über hülsenartige Zwischenelemente 206, 207 und 208 mit beidseitiger Stirnverzahnung, die als Sterilbarriere fungieren. Die Zwischenhülsen 206, 207, 208 sind durch die Halteringe 209, 210, 211 untereinander und mit der Sterilbarriere 5 verbunden. Auch die Innendurchführung bzw. das sterile Führungsrohr 27 ist auf diese Weise mit der innersten Zwischenhülse 208 verbunden, sodass die gesamte Anordnung eine Sterilbarriere mit Spaltdichtungen darstellt. Die Zwischenhülsen 206, 207, 208 dienen lediglich der einfachen Handhabung bei der Installation der Sterilbarriere, lassen aber ansonsten alle zur Funktion erforderlichen Bewegungen zu. Sie fungieren als Spalt- bzw. Labyrinthdichtungen.

Die auf den Antriebswellen 10, 13 und 15 angeordneten Schiebehülsen 200, 201, 202 sind jeweils mit den Wellen drehfest, jedoch axial verschieblich verbunden, beispielsweise durch ein Zahn- oder Polygonwellenprofil. Somit kann die zur Kraftübertragung nötige axiale Vorspannkraft beispielsweise durch Federn, die auf die Schiebehülsen 200, 201, 202 wirken, aufgebracht werden. Zugleich wird ein möglicher Axialversatz zwischen den antriebs- und instrumentenseitigen Wellenabschnitten ausgeglichen.

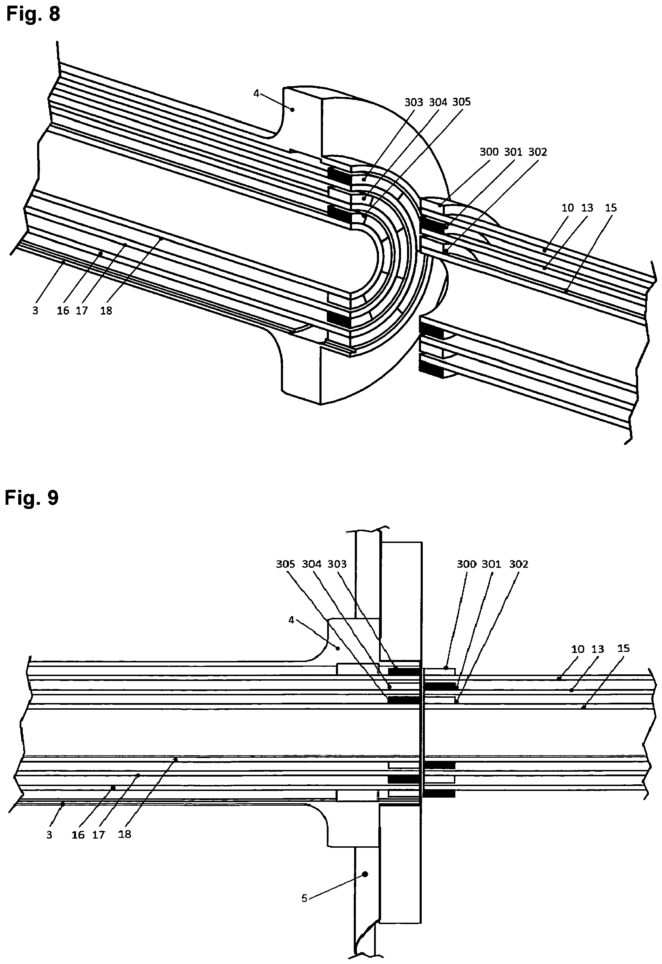

Eine weitere Variante einer Wellenkupplung mit Sterilbarriere ist die in Fig. 8 gezeigte Magnetkupplung. Die Wellenkupplung kann anstelle der Kegel- bzw. Wellenkupplung der Fig. 4 bis 7 insbesondere bei einer Instrumentenanordnung nach Fig. 1 oder 2, 3 vorgesehen sein.

An den distalen Enden der Antriebswellen 10, 13 und 15 sind jeweils Koppelteile in Form von Magnetringen 200, 201 bzw. 202 fixiert. Analog sind auf den instrumentenseitigen Hohlwellen 16, 17 und 18 jeweils Koppelteile in Form von Magnetringen 203, 204 bzw. 205 fixiert. Alle Magnetringe 200 bis 205 sind sektorweise magnetisiert und mit, vorzugsweise kleinem, Axialabstand bzw. Luftspalt zueinander ausgerichtet, um möglichst hohe Antriebsmomente übertragen zu können.

Die Höhe des übertragbaren Moments hängt neben dem Luftspalt auch von der magnetischen Feldstärke und der Anzahl der Magnetsektoren ab.

Fig. 9 zeigt die Magnetkupplung mit Sterilbarriere in einem Schnitt. Die Magnetringe sind mit minimalem Axialabstand zueinander ausgerichtet, um möglichst hohe Antriebsmomente übertragen zu können. Ein vorteilhaftes Merkmal dieses Kupplungsprinzips ist die einfache Gestaltung der sterilen Hülle 5. Aufgrund des geringen axialen Luftspalts der Magnetkupplung kann eine einfache Folie verwendet werden und erfordert kein spezielles Formteil.

### Umsetzen der Bewegungsart Rotation-Translation auf Instrumentenseite

In einer Ausführung der vorliegenden Erfindung werden ausschließlich Drehantriebe verwendet. Die Antriebsstränge in robotergeführten chirurgischen Instrumenten verwenden jedoch aufgrund des engen Bauraums im Instrumentenschaft überwiegend Seilzüge oder Zug-/Druckstangen zur Übertragung der Antriebsbewegungen zum distalen Ende des Instruments. Daher ist nach vorstehend beschriebener trennbarer Instrumenten-Schnittstelle ein instrumentenseitiges Umsetzgetriebe 22 vorgesehen, um die rotatorische Antriebsbewegung in eine translatorische Bewegung der Seilzüge oder Zug-/Druckstangen zu wandeln.

Fig. 10 zeigt in zwei perspektivischen Ansichten ein Umsetzgetrieb 22 nach einer Ausführung der vorliegenden Erfindung, bei dem für jede Antriebswelle eine eigene Schiebehülse vorgesehen ist. Im hier dargestellten Fall setzen die drei Schiebehülsen 23, 24 und 25 die Drehung der Antriebswellen 16, 17 und 18 in eine Translation der Zug- und/oder Schubmittel 26, 39 und 40 um. Die Schiebehülsen 23, 24 und 25 fungieren zugleich als distales Loslager für die Hohlwellen 16, 17 bzw. 18. Die Schiebehülsen selbst verfügen nur über einen translatorischen Freiheitsgrad, der die Verschiebung entlang der Schaftachse zulässt. Die Einschränkung der Freiheitsgrade der Schiebehülsen 23, 24 und 25 wird durch Nut-Führungen 41, 42, 43 erreicht, die ineinander geschachtelt sind. Die Schiebehülsen 23, 24 und 25 sind derart ineinander gesteckt, dass eine außen liegende Hülse die Lagerung der innen liegenden Hülse übernimmt. Dadurch wird eine sehr kompakte Bauform erreicht.

Entsprechend ist die äußere Schiebehülse 23 im Instrumentenschaft 3 gelagert. Eine Übergangspassung zwischen der Hülse 23 und dem Schaft 3 dient als Radiallagerung.

Eine Drehung der Hülse 23 wird durch eine an der Hülse 23 fixierte Passfeder 41 blockiert, die in einer Nut, die in den Instrumentenschaft 3 eingebracht ist, gleitet. Die Schiebehülse 24 ist in der äußeren Schiebehülse 23 gelagert. Eine Übergangspassung zwischen Hülse 24 und Hülse 25 dient als Radiallagerung. Eine Drehung der Hülse 24 wird durch die Nutführung 42 blockiert. Die innere Schiebehülse 25 ist in der Schiebehülse 24 gelagert. Eine Übergangspassung zwischen Hülse 25 und Hülse 24 dient als Radiallagerung. Eine Drehung der Hülse 25 wird durch die Nutführung 43 blockiert.

Die Ankopplung der Antriebswellen an die Schiebehülsen erfolgt mit einer Nutführung, deren Funktionsweise exemplarisch mit Bezug auf die innenliegende Hohlwelle 18 und Fig. 11 erläutert wird, die einen vergrößerten Schnitt zeigt. Am distalen Ende von Hohlwelle 18 ist eine wendelförmige Nut 37 eingebracht. Ein Stift 38, der an der Schiebehülse 25 fixiert ist, greift formschlüssig in die Wendelnut 37 ein. Somit führt eine Drehung der Hohlwelle 18 zu einer Verschiebung der Hülse 25 entlang der Schaftachse und somit auch zu einer Verstellbewegung des Zug- und/oder Schubmittels 26. Am distalen Ende der Schiebehülsen 23, 24 und 25 sind die Zug- und/oder Schubmittel 26, 39 und 40 angebunden, die die Antriebsbewegung zu den Instrumentenfreiheitsgraden bzw. einem Endeffektor am distalen Ende des Instrumentenschafts 3 übertragen.

Ein Vorteil dieser Lösung ist, dass die Antriebsbewegungen, insbesondere Stellwinkel und Winkelgeschwindigkeit, innerhalb eines jeden Instruments an die jeweiligen Erfordernisse angepasst werden können, da die Steigung der Kulissenführung das Übersetzungsverhältnis und den Arbeitsbereich bestimmt. Somit kann die Antriebseinheit für eine möglichst große Anzahl verschiedenartiger Instrumente eingesetzt und die Wirtschaftlichkeit und Benutzerfreundlichkeit gesteigert werden.

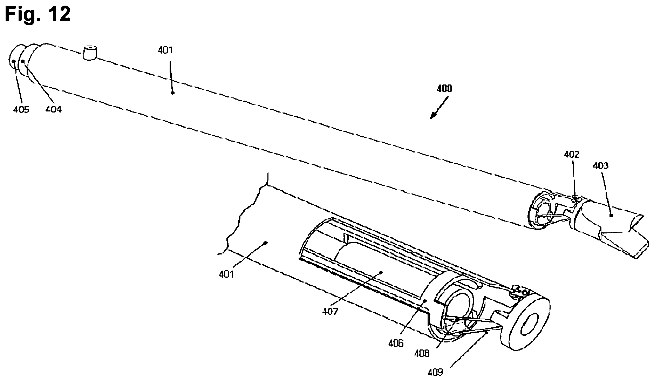

Anstelle der in Fig. 10, 11 dargestellten proximalen Anordnung kann das Umsetzgetriebe alternativ auch am distalen Ende des Instruments, also möglichst nahe an der Instrumentenkinematik und dem Endeffektor angeordnet werden. Fig. 12 zeigt ein Instrument 400 nach einer Ausführung der vorliegenden Erfindung mit einem distalen Umsetzgetriebe in einer perspektivischen Gesamtansicht (oben in Fig. 12) bzw. einem vergrößerten Teilschnitt (unten in Fig. 12).

Das Umsetzgetriebe befindet sich am distalen Ende des Instruments 400 und damit nahe an der Instrumentenkinematik 402 und dem Endeffektor 403. Die Aktuation des distalen Gelenks 402, das im gezeigten Beispiel als Parallelkinematik ausgeführt ist, geschieht mit Zug- und/oder Schubmitteln in Form von Koppelstangen 408 und 409, die mit dem Segment, das den Endeffektor trägt, drehbar verbunden sind. Die jeweils anderen Enden der Koppelstangen 408 und 409 sind mit den Schiebehülsen 406 und 407 drehbar verbunden, die zum Einstellen der Gelenkwinkel entlang der Schaftachse verfahren werden. Die Schiebehülsen 406 und 407 sind mit den Hohlwellen 404 bzw. 405 verbunden, wobei die Umsetzung der rotatorischen Antriebsbewegung in die translatorische Vorschubbewegung der Hülse mit der mit Bezug auf Fig. 10, 11 beschriebenen Kulissenmechanik erfolgt. Im Zentrum der inneren Hohlwelle 405 verbleibt ausreichend Platz, um Antriebsmittel, beispielsweise einen Bowdenzug oder eine Drehwelle mit biegsamem Abschnitt im Bereich des Mehrfachgelenks zum Antrieb des Endeffektors 403 und/oder ein Zusatz-Instrument, insbesondere elektrische Zuleitungen, Schläuche oder dergleichen durchzuführen.

Im Gegensatz zu den bei üblichen Instrumenten der minimal-invasiven Roboterchirurgie verwendeten Seilzügen wird die Antriebsleistung von der Antriebseinheit zur Instrumentenspitze bei dieser Ausführung mit zum Instrumentenschaft koaxialen Hohlwellen übertragen. Daraus kann eine deutlich höhere Belastbarkeit und Steifigkeit des Antriebsstrangs gegenüber Seilzügen oder dünnen Vollwellen resultieren, weshalb vorteilhafterweise höhere Antriebskräfte übertragen werden können. Somit empfiehlt sich diese Ausführung besonders für Instrumente, bei denen höhere Prozesskräfte auftreten, z.B. Klammernahtgeräte.

### Sterilbarriere zwischen Antriebseinheit und Instrument

Einige Komponenten der Antriebseinheit halten den Umweltbedingungen während eines Sterilisationsprozesses nicht stand. Deshalb weist die Instrumenten-Schnittstelle eine sterile Hülle auf, die die Antriebseinheit im Betrieb abdeckt. Neben der Hülle, die das Gehäuse der Antriebseinheit sicher umschließt und üblicherweise als Folienschlauch ausgeführt ist, soll die Instrumenten-Schnittstelle zwischen Antriebseinheit und Instrument die Übertragung von mechanischer Leistung und elektrischen Signalen ermöglichen und zugleich die Kontamination des Operationsfeldes durch die unsterile Antriebseinheit verhindern.

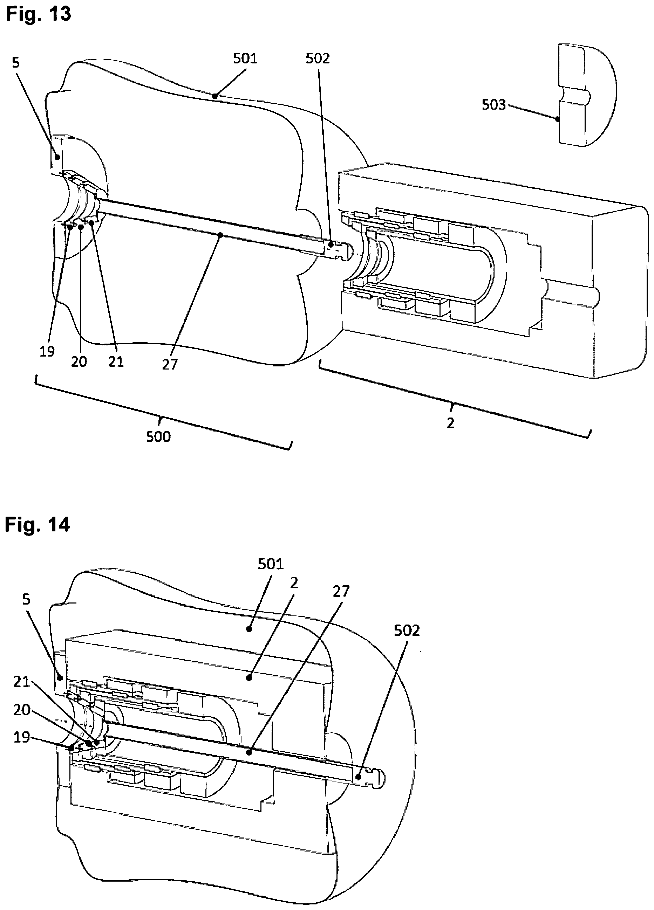

Fig. 13 zeigt einen Überblick der Instrumenten-Schnittstelle 500 mit verschiedenen Teilkomponenten, die beispielsweise bei der Instrumentenanordnung der Fig. 1 vorgesehen sein kann.

Die Instrumenten-Schnittstelle 500 weist eine sterile Folienhülle 501 auf, die das Gehäuse der Antriebseinheit 2 umschließt, einen formstabilen Flansch bzw. Instrumententräger 5, der zur Koppelung der Antriebsstränge beispielsweise die mit Bezug auf Fig. 4 beschriebenen kegelförmigen Zwischenelementen 19, 20, 21 aufweist, sowie eine Innendurchführung in Form des Führungsrohrs 27. Ein Abschlussring 503 verbindet das Führungsrohr 27 mit der Folienhülle 501. Um die Sterilität des Führungsrohrs 27 beim Einführen in die Antriebseinheit 2 zu wahren, ist das Führungsrohr 27 an seinem proximalen Ende zunächst mit einem Blindstopfen 502 verschlossen, der einen Teil der Mantelfläche mit abdeckt. Die Instrumenten-Schnittstelle 500 ist als Komplett-Baugruppe konzipiert, bei der alle Einzelteile zu einer Einheit verbunden sind. Dadurch wird die Handhabung stark vereinfacht. Im Falle der mit Bezug auf Fig. 8 erläuterten Magnetkupplung ist eine Folie als Sterilbarriere bzw. Instrumenten-Schnittstelle zwischen den Wellenabschnitten ausreichend.

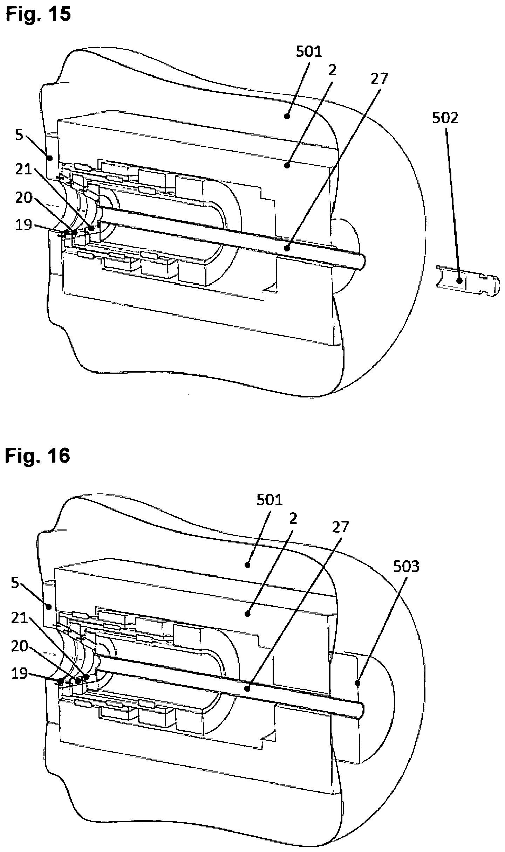

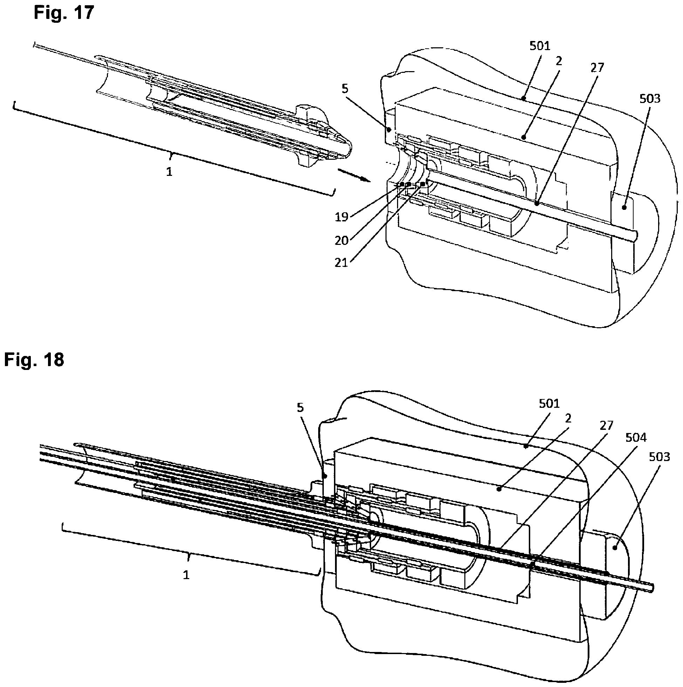

Die weiteren Fign. 14 bis 17 veranschaulichen das sterile Einpacken der Antriebseinheit und den Anschluss eines chirurgischen Instruments an diese. Als erster Schritt (vgl. Fig. 14) wird der Instrumententräger 5 auf die Antriebseinheit gesetzt. Zugleich wird das sterile Führungsrohr 27 in die Hohlwelle sowie die Zwischenstücke 19, 20, 21 der Wellenkupplungen in die Antriebseinheit 2 eingeführt und die Folienhülle über die Antriebseinheit 2 gestreift. Dann wird der Blindstopfen 502, der nach dem Durchschieben des sterilen Führungsrohrs 27 durch die Hohlwelle der Antriebseinheit 2 unsteril ist, durch einen unsterilen Mitarbeiter des OP-Teams vom Führungsrohr 27 abgezogen und entsorgt (vgl. Fig. 15). Da der Blindstopfen 502 auch einen Teil der Mantelfläche des Führungsrohrs 27 abdeckt, bleibt der Abschnitt des Führungsrohrs 27, der aus der Antriebseinheit 2 ragt, steril. Schließlich wird die sterile Hülle durch Fügen des Abschlussrings 503 auf das Führungsrohr 27 abgeschlossen (vgl. Fig. 16). Fig. 17 zeigt schließlich das Andocken eines chirurgischen Instruments 1 an die steril verpackte Antriebseinheit 2.

### Führen von zusätzlichen Antriebssträngen und/oder Zusatz-Instrumenten durch den Instrumentenschaft zum distalen Ende des Instruments

Neben der einfachen mechanischen Gestaltung der trennbaren Instrument-Schnittstelle bietet die koaxiale Anordnung aller Antriebswellen den Vorteil, dass das Zentrum von Antriebseinheit und Instrument frei sind, um zusätzliche Antriebsmittel, zum Beispiel Seilzüge, Bowdenzüge und/oder Drehwellen, zum Betätigen des Endeffektors durchzuführen. Zum Beispiel kann ein Bowdenzug doppelt verwendet werden: Die Hülle dient der Übertragung einer ersten, die Seele der Übertragung einer weiteren Betätigungskraft. Es können auch elektrische Leitungen für Monopolar- oder Bipolarinstrumente, oder Saug- und Spülschläuche in der Mitte des Instrumentenschafts geführt werden. Gleichermaßen können auch andere Zusatz-Instrumente durch den Roboter geführt werden, zum Beispiel Lichtwellenleiter für Laseranwendungen oder flexible Instrumente zur Argon-Plasma Koagulation, für die Kryo-Chirurgie oder Wasserstrahl-Chirurgie, die häufig für Tumorresektionen eingesetzt werden.

Fig. 18 zeigt eine Instrumentenanordnung mit einem ein- bzw. durchgeführten starren oder flexiblen Zusatz-Instrument.

Hierzu wird das Zusatz-Instrument 504 nach Anlegen der sterilen Hülle 501 durch das Führungsrohr 27 von hinten durch die Antriebseinheit 2 bis zum distalen Ende des Instruments 1 vorgeschoben und in dieser Lage fixiert. Anschließend kann das Zusatz-Instrument 504 wie ein gewöhnliches robotergeführtes Instrument verwendet werden und mit den vom Instrument 1 bereitgestellten Freiheitsgraden im Operationsgebiet bewegt werden. Neben der Eignung für starre und flexible Zusatz-Instrumente ist ein Vorteil dieser Lösung, dass kein zusätzlicher Bauraum im Bereich der trennbaren Instrument-Schnittstelle benötigt wird, um das Zusatz-Instrument 504 in den Instrumentenschaft einzuführen.

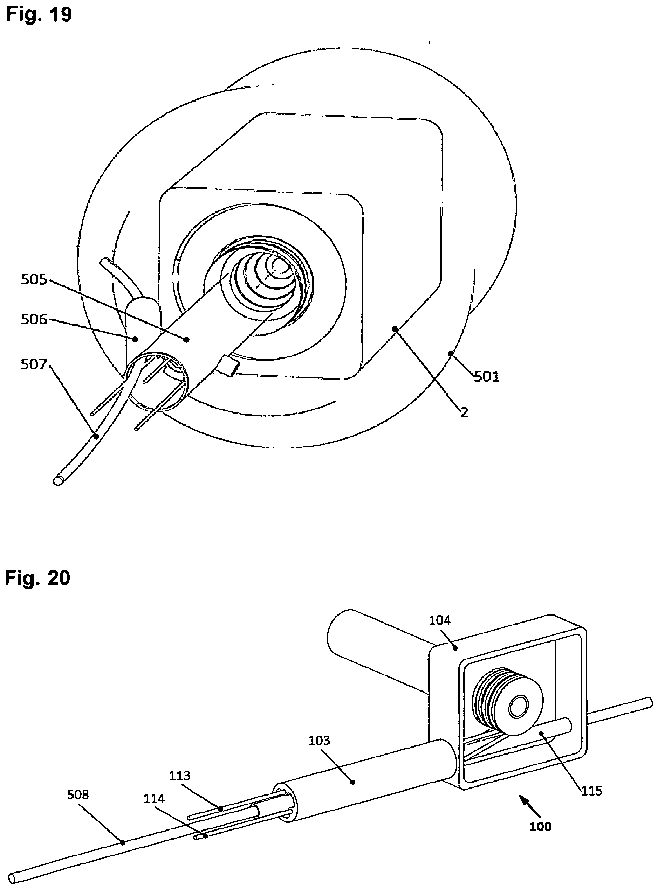

Fig. 19 zeigt einen Teil eines Instruments einer Instrumentenanordnung nach einer weiteren Ausführung der vorliegenden Erfindung, die sich insbesondere für flexible Zusatz-Instrumente eignet. Hier wird das Zusatz-Instrument 507 nicht durch die Antriebseinheit 2, sondern durch einen gebogenen Rohrabschnitt 506 eingeführt, der am Instrumentenschaft 505 unmittelbar vor der Antriebseinheit 2 bzw. Instrumenten-Schnittstelle angeordnet ist. Bei dieser Lösung kann die sterile Hülle 501 einfacher gestaltet sein, da das Zusatz-Instrument 507 nicht von hinten durch die Antriebseinheit geführt wird.

Fig. 20 zeigt einen Teil eines Instruments einer Instrumentenanordnung nach einer weiteren Ausführung der vorliegenden Erfindung, bei der Antriebs- und Schaftachse orthogonal sind. Hier können sowohl starre als auch flexible Zusatz-Instrumente ein- bzw. durchgeführt werden. Das Zusatz-Instrument 508 wird durch ein Führungsrohr 115 von hinten durch das Gehäuse 104 bis zum distalen Ende des Instrumentenschafts 103 vorgeschoben und fixiert. Anschließend kann das Zusatz-Instrument 508 wie ein gewöhnliches robotergeführtes Instrument verwendet werden und mit den vom Instrument 100 bereitgestellten Freiheitsgraden im Operationsgebiet bewegt werden. Auch hier ist kein Bauraum am proximalen Ende des Instrumentenschafts nötig, um das Zusatz-Instrument 508 einzuführen.

Die Antriebseinheit stellt die mechanische Antriebsleistung für alle aktiven Freiheitsgrade des chirurgischen Instruments zur Verfügung. Sie befindet sich am proximalen Ende des Instruments und ist als eigenständiges Modul konzipiert, das sich als Antrieb unterschiedlicher Instrumente eignet. Um eine Kontamination des Operationsgebiets zu vermeiden, ist die Antriebseinheit mit einer sterilen Schutzhülle hermetisch abgeschlossen.

Die trennbare Instrumenten-Schnittstelle befindet sich zwischen der Antriebseinheit und dem chirurgischen Instrument. Ihre primäre Aufgabe ist die mechanische Anbindung des chirurgischen Instruments an die Antriebseinheit. Sie stellt zum einen den Kraftfluss zwischen den Funktionseinheiten Antrieb und Instrument her und sorgt für eine exakte und wiederholgenaue Relativ-Positionierung und Fixierung dieser Einheiten. Um die benötigte mechanische Leistung auf das Instrument zu übertragen, umfasst die trennbare Instrumenten-Schnittstelle außerdem lösbare Kupplungen, die den Kraftfluss zwischen den Einzelantrieben in der Antriebseinheit und den Antriebssträngen im Instrument herstellen. Um die Sterilität des chirurgischen Instruments unter allen Umständen zu gewährleisten, fungiert die trennbare Instrumenten-Schnittstelle zugleich als Sterilbarriere zwischen der unsterilen Antriebseinheit und einem sterilen Instrument.

Vorteilhafterweise ist das Ankoppeln eines chirurgischen Instruments einer Instrumentenanordnung nach einer Ausführung der vorliegenden Erfindung einfach und erfordert keine vertieften Fachkenntnisse des Robotersystems. Die trennbare Instrumenten-Schnittstelle nach einer Ausführung erlaubt vorteilhafterweise das wiederholbar zuverlässige Einkoppeln des Instruments einschließlich aller Kraftübertragungselemente ohne visuelle Kontrolle. Die Schnittstelle kann vorzugsweise eine oder mehrere Antriebsbewegungen von einer Antriebseinheit auf ein chirurgisches Instrument übertragen, während die Sterilität auf Instrumentenseite gewahrt bleiben kann. Antriebseinheit und/oder trennbare Instrumenten-Schnittstelle beanspruchen vorteilhafterweise einen geringen Bauraum, um insbesondere bei einem System mit mehreren Robotern die Kollisionsgefahr zu minimieren. Um die Leistungsfähigkeit des robotergeführten Instruments aus regelungstechnischer Sicht zu verbessern, ist die Übertragung von mechanischer Antriebsenergie auf das chirurgische Instrument möglichst spiel- und schlupffrei ausgebildet.

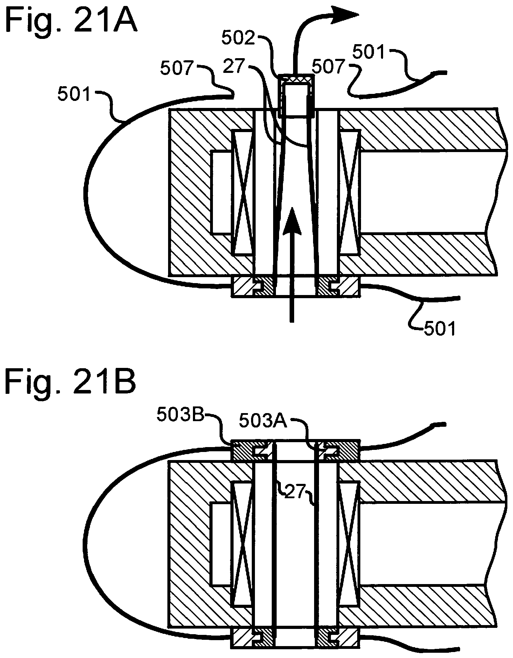

Fig. 21A, 21B zeigen ein Umhüllen eines Roboters mit einer Hülle nach einer Ausführung der vorliegenden Erfindung. Der Roboter, dessen Roboterhand in Fig. 21A, 21B teilweise angedeutet ist, weist eine Hohlwelle auf. Durch diese wird eine schlauchartige Innendurchführung 27 einer Hülle 501 geführt, die eine Austrittsöffnung 507 aufweist. Das durchgeführte Ende der Innendurchführung 27 ist anfangs, beispielsweise klemmschlüssig, mit einem Blindstopfen 502 abgedeckt, der eine geschlossene Stirnfläche und eine rohrartige Mantelfläche aufweist, um das Innere und einen stirnseitigen Umfangsbereich der Innendurchführung 27 vor Verschmutzung beim Durchführen zu schützen.

Der Blindstopfen 502 wird durch die Hohlwelle und die Austrittsöffnung 507 durchgeführt (Fig. 21A) und anschließend entfernt. An dem, dadurch freigewordenen Umfangsbereich der Innendurchführung 27 und dem Rand der Austrittsöffnung 507 wird, wiederum beispielsweise klemmschlüssig, ein steriler Abschlussring 503A, 503B befestigt (Fig. 21B). So kann in einfacher Weise eine sterile Umhüllung des Roboters mit Hohlwelle bzw. gleichermaßen der Antriebseinheit einer Instrumentenanordnung dargestellt werden.

Im Ausführungsbeispiel ist die Innendurchführung 27 an ihrem dem Blindstopfen 502 bzw. Abschlussring 503 entgegengesetzten Ende (unten in Fig. 21A, 21B) drehhab an der Hülle 501 gelagert, beispielsweise wie vorstehend mit Bezug auf die Zwischenelemente der Instrumenten-Schnittstelle beschrieben. Der Abschlussring ist zweiteilig ausgebildet, wobei ein an dem Umfangsbereich der Innendurchführung befestigter Teil 503A des Abschlussringes drehbar an einem an der Austrittsöffnung der Hülle befestigten Teil 503B des Abschlussringes gelagert ist. Auf diese Weise ist die Innendurchführung 27 als ganzes drehbar an der Hülle 501 gelagert und kann insbesondere mit einem durch die Hohlwelle bewegten Zusatz-Instrument mitgedreht werden. In einer (nicht dargestellten) Abwandlung kann die Innendurchführung auch integral bzw. einstückig mit der Hülle ausgebildet und/oder mittels eines einteiligen Abschlussringes verbunden sein, wobei eine eventuelle Drehung der Hohlwelle beispielsweise durch Lose der Innendurchführung bzw. Hülle kompensiert werden kann.

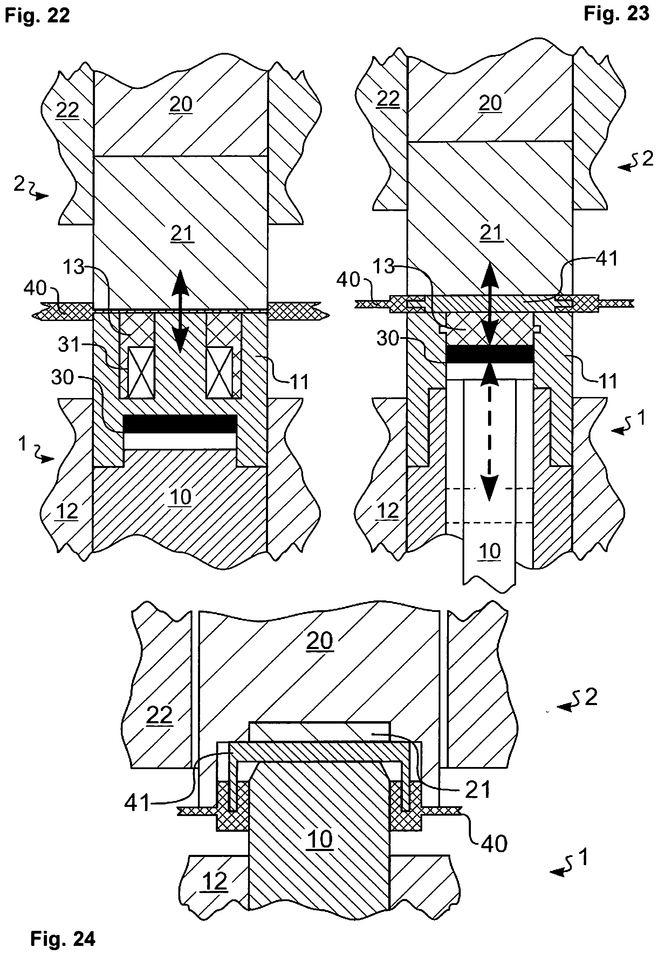

Fig. 22 zeigt einen Teil eines minimalinvasiven chirurgischen Instruments nach einer Ausführung der vorliegenden Erfindung in einem Längsschnitt mit einem Instrumentenmodul 1 und einem damit lösbar verbundenen Instrumententeil 2.

Das Instrumententeil weist einen in einen Patienten einführbaren Instrumentenschaft 22 mit einem Endeffektor auf (nicht dargestellt), das Instrumentenmodul einen Antrieb zum Aktuieren des Endeffektors sowie eine elektromechanische Schnittstellung zur Befestigung an einem Roboter (nicht dargestellt).

Das Instrumentenmodul 1 weist eine Koppelelementanordnung mit mehreren Koppelelementen in Form von translatorisch beweglichen Stößeln 10 auf, die in einem Schublager 12 des Instrumentenmoduls verdrehsicher verschiebbar geführt sind und von denen in Fig. 22 zur besseren Übersicht nur eines dargestellt ist. Das Instrumententeil weist eine entsprechende Gegenelementanordnung mit Gegenelementen in Form von translatorisch beweglichen (Gegen)Stößeln 20 auf, die in einem Schublager des Instrumentenschafts 22 verdrehsicher verschiebbar geführt sind, um jeweils einen Freiheitsgrad des Endeffektors zu aktuieren. Die translatorische Bewegung von Stößel und Gegenstößel zum Aktuieren eines intrakorporalen Freiheitsgrades des minimalinvasiven Instruments durch den extrakorporalen Antrieb ist in Fig. 22 durch einen Bewegungsdoppelpfeil angedeutet.

Die Stößel 10 der Koppelelementanordnung sind mit den Gegenstößeln 20 der Gegenelementanordnung magnetisch koppelbar. Hierzu weisen die Stößel 10 jeweils eine Magnetanordnung zum magnetischen Ankoppeln des gegenüberliegenden Gegenstößels 20 auf, der einen magnetisch beaufschlagbaren Bereich 21 aus einem ferromagnetischen oder dauermagnetischen Material aufweist. Die Stößel 10 der Koppelelementanordnung weisen einen magnetisch leitenden Bereich 11 aus einem ferromagnetischen Material auf, der einen Außenring und ein zentrales Joch aufweist.

Um dieses Joch ist eine elektrische Spule angeordnet und mit einer nichtmagnetischen Vergussmasse 13 vergossen, um integral mit dem Stößel 10 einen Elektromagneten 31 der Magnetanordnung auszubilden, der durch ein hierzu eingerichtetes Steuermittel, das in einer Antriebssteuerung des Instruments (nicht dargestellt) implementiert ist, wahlweise bestrombar ist bzw. bestromt wird.

Zusätzlich weist jede Magnetanordnung einen zu dem Elektromagneten 31 gegensinnigen Permanentmagneten 30 auf, dessen Magnetfeld durch den bestromten Elektromagneten 31 in einem stirnseitigen Koppelbereich von Stößel und Gegenstößel, wenigstens im Wesentlichen, kompensiert wird.

Durch den wahlweise bestrombaren Elektromagneten 31 wird eine stromlos geschlossene Ankopplung zwischen Koppel- und Gegenelement zur Verfügung gestellt: solange der Elektromagnet 31 stromlos ist, koppelt der Permanentmagnet 30 den magnetisch beaufschlagbaren Bereich 21 des Gegenstößels 20 betriebssicher an den magnetisch leitenden Bereich 11 des Stößels 10. Durch Bestromen des Elektromagneten 31 kompensiert dieser das Magnetfeld des Permanentmagneten 30 im stirnseitigen Koppelbereich soweit, dass das Instrumententeil 2, vorzugsweise unter Eigengewicht und/oder geringer Handkraft, von dem Antriebsmodul 1 entfernt werden kann.

Gleichermaßen können bestromter Elektromagnet 31 und Permanentmagnet 30 auch gleichsinnig sein bzw. ihre Magnetfelder sich in einem stirnseitigen Koppelbereich von Stößel und Gegenstößel verstärken.

Zwischen der Koppelelementanordnung und der Gegenelementanordnung ist optional eine sterile Barriere 40 angeordnet, die im Koppelbereich folienartig und flexibel ausgebildet ist, um eine translatorische Bewegung von Stößel 10 und Gegenstößel 20 zur Aktuieren des Endeffektors unter elastischer Deformation mitzumachen.

In einer nicht dargestellten Abwandlung kann der Permanentmagnet 30 entfallen, um durch wahlweises Bestromen des Elektromagneten 31 umgekehrt eine stromlos geöffnete Ankopplung zwischen Koppel- und Gegenelement zur Verfügung zu stellen: solange der Elektromagnet 31 bestromt ist, koppelt er den magnetisch beaufschlagbaren Bereich 21 des Gegenstößels 20 betriebssicher an den magnetisch leitenden Bereich 11 des Stößels 10. Bei stromlosem Elektromagnet 31 kann das Instrumententeil 2 von dem Antriebsmodul 1 entfernt werden.

Fig. 23 zeigt einen Teil eines minimalinvasiven chirurgischen Instruments nach einer weiteren Ausführung der vorliegenden Erfindung in Fig. 22 entsprechender Darstellung. Einander entsprechende Elemente sind durch identische Bezugszeichen bezeichnet, so dass auf die übrige Beschreibung Bezug genommen und nachfolgend nur auf die Unterschiede zur Ausführung der Fig. 22 eingegangen wird.

In der Ausführung der Fig. 23 weist die Magnetanordnung keinen Elektromagneten, sondern nur den Permanentmagneten 30 auf. Insbesondere, um Koppel- und Gegenelement 10, 20 voneinander abzukoppeln, ohne sie voneinander zu beabstanden, ist der Permanentmagnet 30 in dieser Ausführung in dem Koppelelement bzw. Stößel 10 zwischen einer in Fig. 23 dargestellten Verriegelungsposition und einer hiervon beabstandeten, in Fig. 23 strichliert angedeuteten Entriegelungsposition verschiebbar, was in Fig. 23 durch einen strichlierten Bewegungsdoppelpfeil angedeutet ist. Der Permanentmagnet 30 ist in einer Längsbohrung des Stößels 10 verschiebbar geführt und beispielsweise elektromotorisch, hydraulisch, pneumatisch und/oder manuell durch Verschieben einer Schubstange, auf der er angeordnet ist, verstell- und in der Verriegelungs- und der Entriegelungsposition arretierbar.

Der stirnseitige magnetisch leitende Bereich 11 des Stößels 10 wird durch den Permanentmagneten 30, wenigstens im Wesentlichen, nur magnetisch beaufschlagt, wenn dieser in der Verriegelungsposition ist. In der Entriegelungsposition (strichliert in Fig. 23) ist der Permanentmagnet 30 hingegen von dem magnetisch leitenden Bereich 11 des Stößels 10 getrennt und in einem magnetisch nicht leitenden Bereich des Stößels 10 aus Kunststoff mit einer Permeabilitätszahl µᵣ angeordnet, die höchstens 2 beträgt.

Indem der Permanentmagnet 30 in der Bohrung des Stößels 10 in die Verriegelungsposition verstellt wird, kann dessen magnetisch leitender Bereich 11 zum Ankoppeln des Gegenstößels durch die Magnetanordnung wahlweise magnetisch beaufschlagt werden.

In der Ausführung der Fig. 23 weist die optionale sterile Barriere 40 ein steifes Koppelstück 41 aus einem magnetisch leitenden Material auf, um die mechanische Kraftübertragung und magnetische Ankopplung zu verbessern. In einer nicht dargestellten Abwandlung kann die optionale sterile Barriere auch folienartig wie in Fig. 22 ausgebildet sein bzw. die optionale Barriere der Ausführung der Fig. 22 ein solches Koppelstück aufweisen.

In der Ausführung der Fig. 23 ist mit 13 ein Bauteil aus einem nichtmagnetischen Werkstoff bezeichnet. Dieses kann, wie in der Ausführung der Fig. 22, als Vergussmasse ausgebildet sein und ist daher durch dasselbe Bezugszeichen bezeichnet. Gleichermaßen kann das Bauteil 13 ein Formteil sein, welches an dem magnetisch leitenden Bereich 11 befestigt ist und als Anschlag für den verschiebbaren Permanentmagnet 30 fungiert.

Fig. 24 zeigt einen Teil eines minimalinvasiven chirurgischen Instruments nach einer weiteren Ausführung der vorliegenden Erfindung in Fig. 22, 23 entsprechender Darstellung. Einander entsprechende Elemente sind wieder durch identische Bezugszeichen bezeichnet, so dass auf die übrige Beschreibung Bezug genommen und nachfolgend nur auf die Unterschiede zur Ausführung der Fig. 22, 23 eingegangen wird. Insbesondere sind zur besseren Übersicht in Fig. 24 die Magnetanordnung und die magnetisch leitenden Bereiche nicht dargestellt, diese können insbesondere so ausgebildet sein, wie in Fig. 22 oder 23 dargestellt bzw. mit Bezug hierauf erläutert.

In der Ausführung der Fig. 24 sind Stößel 10 und Gegenstößel 20 zusätzlich zu der magnetischen Ankopplung formschlüssig verbindbar bzw. verbunden. Hierzu greift der Stößel 10 bolzenartig in einen Hülsen- bzw. Buchsenbereich des Gegenstößels 20 ein, wenn Koppel- und Gegenelement aneinander gekoppelt sind. Hierdurch sind Koppel- und Gegenelement senkrecht zu ihrer in Fig. 24 vertikalen Längserstreckung, d.h. horizontal in der Zeichenebene bzw. senkrecht auf dieser, formschlüssig festgelegt, wobei die magnetische Kopplung sie in Richtung ihrer Längserstreckung kraftschlüssig festlegt.

Stößel 10 und Gegenstößel 20 sind hierdurch formschlüssig aneinander zentriert. Um dabei einen Lateral- und/oder Winkelversatz zu kompensieren, ist der Gegenstößel 20 in der Ausführung der Fig. 24 mit Spiel in dem Schublager des Instrumentenschafts 22 gelagert.

Der magnetisch leitende Bereich 21 des Gegenstößels 20 ist in der Ausführung der Fig. 24 im Inneren des Hülsen- bzw. Buchsenbereichs des Gegenstößels 20 angeordnet, um so vorzugsweise eine unbeabsichtigte magnetische Interferenz zu vermeiden.

In den erläuterten Ausführungen sind Koppel- und Gegenelement stößelartig ausgebildet und auf Stoß bzw. stirnseitig aneinander gekoppelt, wobei die Magnetanordnung Koppel- und Gegenelement in Richtung ihrer Längserstreckung gegeneinander zieht, um Zugkräfte zu übertragen, während Druckkräfte formschlüssig übertragen werden.

Zusätzlich oder alternativ können in den Ausführungen Koppel- und Gegenelemente 10, 20 rotatorisch beweglich sein, um jeweils einen Freiheitsgrad des Endeffektors zu aktuieren. Die Magnetanordnung zieht Koppel- und Gegenelement in Richtung ihrer Längserstreckung gegeneinander, um in einer Ausführung eine Übertragung von Drehmomenten zu ermöglichen. Diese kann reibschlüssig infolge der axialen Verspannung durch die Magnetanordnung erfolgen. Gleichermaßen kann sie auch formschlüssig erfolgen. Hierzu kann in einer nicht dargestellten Abwandlung der Stößel 10 oder Gegenstößel 20 einen oder mehrere exzentrische Vorsprünge, insbesondere Zähne, aufweisen, die in entsprechende Aussparungen, insbesondere Zahnlücken, in dem Gegenstößel 20 bzw. Stößel 10 eingreifen, wenn Koppel- und dem Gegenelement magnetisch aneinander gekoppelt sind. In einer ebenfalls nicht dargestellten Abwandlung weisen Stößel 10 oder Gegenstößel 20 eine Hirth-Verzahnung auf.

Das Koppelstück 41 kann insbesondere in einem solchen Fall mittels einer Drehdichtung drehbeweglich mit der restlichen Barriere 40 verbunden sein. Gleichermaßen kann das Koppelstück 41 mittels einer translatorischen Dichtung verschiebbar mit der restlichen Barriere 40 verbunden sein, wie jeweils in Fig. 24 angedeutet.

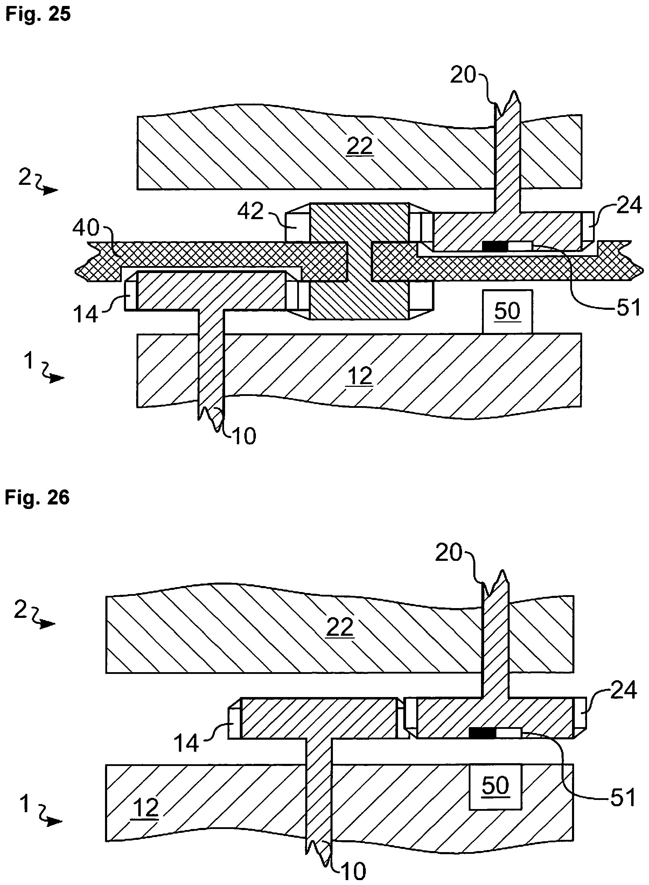

Fig. 25, 26 zeigen jeweils einen Teil eines minimalinvasiven chirurgischen Instruments nach einer weiteren Ausführung der vorliegenden Erfindung in einem Längsschnitt mit einem Instrumentenmodul 1 und ein damit lösbar verbundenes Instrumententeil 2. Einander entsprechende Elemente sind wieder durch identische Bezugszeichen bezeichnet, so dass auf die vorhergehende Beschreibung Bezug genommen und nachfolgend nur auf die Unterschiede zur Ausführung der Fig. 22-24 eingegangen wird.

In der Ausführung der Fig. 26 sind ein Koppelelement in Form einer drehbar gelagerten Abtriebswelle 10 eines Elektromotors des Antriebs (nicht dargestellt) und ein Gegenelement in Form einer dazu parallel versetzt drehbar gelagerten Antriebswelle 20 eines Endeffektors des Instruments (nicht dargestellt) durch miteinander kämmende Stirnräder 14, 24 formschlüssig aneinander gekoppelt. In der Ausführung der Fig. 25 ist zwischen Koppel- und Gegenelementanordnung eine sterile Barriere 40 mit einem drehbeweglich gelagerten Koppelstück 42 mit zwei Stirnrädern angeordnet, die mit den Stirnrädern 14 bzw. 24 kämmen und so Koppel- und Gegenelement 10, 20 ebenfalls formschlüssig koppeln.

Mit 12 bzw. 22 ist jeweils ein Drehlager bzw. Gehäuse des Instrumentenmoduls 1 bzw. Instrumententeils 2 angedeutet.

In einer nicht dargestellten Abwandlung können Koppel- und Gegenelement 10, 20 zusätzlich oder alternativ magnetisch miteinander drehfest gekoppelt sein, wie dies vorstehend mit Bezug auf Fig. 22-24 erläutert wurde.

Die Stirnverzahnungen zwischen den Stirnrädern 14, 24 und gegebenenfalls 42 sind mehrdeutig bzw. in unterschiedlichen, um die Zahnteilung versetzten Orientierungen aneinander koppelbar.

Um gleichwohl den Endeffektor durch einen Antrieb ohne vorherige Rekalibrierung aktuieren zu können, weist das Instrumentenmodul 1 in den Ausführungen der Fig. 25, 26 einen berührungslosen Winkelsensor in Form eines magnetischen Encoders zum Erfassen der Winkelstellung der angekoppelten Antriebswelle 20 relativ zum Gehäuse bzw. Drehlager 12 des Instrumentenmoduls lauf. Die Antriebswelle 20 weist entsprechend einen drehfesten Sender in Form eines Permanentstabmagneten 51 auf, der dazu eingerichtet ist, durch den Winkelsensor 50 erfasst zu werden. Die Nord-Süd-Achse des Stabmagneten 51 ist senkrecht zur Drehachse der Antriebswelle 20 orientiert.

Beim oder nach dem Ankoppeln des Instrumententeils 2 an das Instrumentenmodul 1 in einer von mehreren Orientierungen wird die Winkelstellung des Senders 51 in der angekoppelten Antriebswelle 20, von denen in Fig. 25, 26 wiederum nur eine exemplarisch dargestellt ist, relativ zu einer instrumentenmodulgehäusefesten Referenz durch den Winkelsensor 50 des Instrumentenmoduls erfasst. Auf diese Weise ist nach dem Erfassen die Orientierung der Gegenelemente und damit auch eine Stellung des Endeffektors bekannt, so dass der Endeffektor durch einen Antrieb ohne Rekalibrierung korrekt aktuiert werden kann.

### Bezugszeichenliste

### In den Figuren 1 bis 21:

- 1, 100, 101, 400: Instrument
- 2, 102: Antriebseinheit
- 4: Anschlussflansch
- 3, 103, 505: Instrumentenschaft
- 5, 105, 501: (sterile) Hülle
- 6, 104: Gehäuse
- 7, 8, 9: Elektromotor
- 11, 12, 14, 28, 29, 30: Lagerstelle
- 19, 20, 21, 206, 207, 208: Zwischenelement
- 22: Umsetzungsgetriebe
- 23, 24, 25, 37, 38, 200, 201, 202, 406, 407: Schiebehülse (Kulissenführung)
- 26, 39, 40: Zug-/Schubmittel
- 31-36, 200-205, 300-305: Stirnverzahnung (Koppelteil)
- 27, 115: (steriles) Führungsrohr
- 37: Wendelnut
- 38: Stift
- 41, 42 ,43: Nut-Führung
- 10, 13, 15, 16, 17, 18, 106, 107, 108, 109, 110, 111, 404, 405, 406: Antriebswelle
- 402: Instrumentenkinematik
- 403: Endeffektor
- 408, 409: Koppelstange
- 100: instrumentenseitiges Umsetzungsgetriebe
- 112, 113, 114: Seilzüge
- 115: Führungsrohr
- 116, 117, 118: Kupplungszwischensegment
- 209, 210, 211: Haltering
- 500: Instrumenten-Schnittstelle
- 501: (sterile) Folienhülle
- 502: Blindstopfen
- 503: Abschlussring
- 504, 507, 508: Zusatz-Instrument
- 506: Rohrabschnitt
- 507: Austrittsöffnung

### In den Figuren 22 bis 26:

- 1: Instrumentenmodul
- 10: Stößel; Welle (Koppelelement)
- 11: magnetisch leitender Bereich
- 12: Instrumentenmodulgehäuse, Schub-/Drehlager
- 13: Vergussmasse; Form-/Bauteil
- 14: Stirnrad
- 2: Instrumententeil
- 20: Stößel; Welle (Gegenelement)
- 21: magnetisch beaufschlagbarer Bereich
- 22: Instrumentenschaft, Schub-/Drehlager
- 24: Stirnrad
- 30: Permanentmagnet
- 31: Elektromagnet
- 40: sterile Barriere
- 41,42: Koppelstück
- 50: Winkelsensor
- 51: Permanentstabmagnet (Sender)

## Patentansprüche

1. Hülle mit einer schlauchartigen Innendurchführung zum Durchführen durch eine Hohlwelle eines Roboters oder einer Antriebseinheit für eine Instrumentenanordnung nach einem der Ansprüche 5 bis 14 mit wenigstens einem Drehantrieb (7, 8, 9) mit einer Antriebswelle, insbesondere Antriebs-Hohlwelle (10, 13, 15; 106, 107, 108), mit einem Koppelteil (31, 32, 33; 200, 201, 202; 300, 301, 302) zur Koppelung mit einer Antriebswelle des Instruments und
durch eine Austrittsöffnung (507) der Hülle,
**dadurch gekennzeichnet, dass**
die Innendurchführung einen Blindstopfen (502), welcher eine rohrartige Mantelfläche aufweist, an einem Ende der Innendurchführung befestigt ist und einen Umfangsbereich der Innendurchführung abdeckt, und einen Abschlussring (503) zur Befestigung an dem äußeren Umfangbereich der Innendurchführung aufweist, der durch Entfernen des Blindstopfens frei wird.

2. Instrumenten-Schnittstelle für eine Instrumentenanordnung nach einem der Ansprüche 5 bis 14, **gekennzeichnet durch** eine Hülle (5, 501) nach Anspruch 1, zum Umhüllen der Antriebseinheit der Instrumentenanordnung.

3. Instrumenten-Schnittstelle nach Anspruch 2, **dadurch gekennzeichnet, dass** die Hülle (5, 501) eine Innendurchführung (27) für ein durch das Instrument und die Antriebseinheit der Instrumentenanordnung eingeführtes Zusatz-Instrument aufweist.

4. Instrumenten-Schnittstelle nach einem der Ansprüche 2 bis 3, **gekennzeichnet durch** wenigstens ein drehbares, insbesondere kegelförmiges, Zwischenelement (19, 20, 21; 206, 207, 208) zur reib- oder formschlüssigen Koppelung mit dem Koppelteil einer Antriebswelle der Antriebseinheit und/oder des Instruments.

5. Instrumentenanordnung zur, insbesondere lösbaren, Befestigung an dem Roboter eines Chirurgierobotersystems nach Anspruch 15, mit:
einer Antriebseinheit (2; 102) mit wenigstens einem Drehantrieb (7, 8, 9) mit einer Antriebswelle, insbesondere Antriebs-Hohlwelle (10, 13, 15; 106, 107, 108), mit einem Koppelteil (31, 32, 33; 200, 201, 202; 300, 301, 302) zur Koppelung mit einer Antriebswelle des Instruments;
einem Instrument (1; 100; 400) mit einem Instrumentenschaft (3) und wenigstens einer Antriebswelle, insbesondere Antriebs-Hohlwelle (16, 17, 18; 109, 110, 111; 404, 405, 406), mit einem Koppelteil (34, 35, 36; 203, 204, 205; 303, 304, 305) zur Koppelung mit einer Antriebswelle der Antriebseinheit, das mit der Antriebseinheit lösbar verbunden ist; und
einer Instrumenten-Schnittstelle (5, 500) nach einem der Ansprüche 2 bis 4, die zwischen Antriebseinheit und Instrument angeordnet ist.

6. Instrumentenanordnung nach Anspruch 5, **gekennzeichnet durch** ein Zusatz-Instrument (504) und/oder ein Antriebsmittel zum Betätigen eines Endeffektors, welches, insbesondere lösbar, durch das Instrument der Instrumentenanordnung eingeführt ist.

7. Instrumentenanordnung nach einem der Ansprüche 5 bis 6, **dadurch gekennzeichnet, dass** wenigstens eine Antriebswelle (10, 13, 15; 106, 107, 108) der Antriebseinheit und eine Antriebswelle (16, 17, 18; 109, 110, 111; 404, 405, 406) des Instruments koaxial, insbesondere fluchten angeordnet sind.

8. Instrumentenanordnung nach einem der Ansprüche 5 bis 7, **dadurch gekennzeichnet, dass** ein Koppelteil (300, 301, 302) einer Antriebswelle der Antriebseinheit und ein Koppelteil (303, 304, 305) einer Antriebswelle des Instruments magnetisch miteinander gekoppelt sind.

9. Instrumentenanordnung nach einem der Ansprüche 5 bis 8, **dadurch gekennzeichnet, dass** die Antriebseinheit wenigstens eine innere Antriebswelle, insbesondere Antriebs-Hohlwelle, aufweist, die konzentrisch in einer äußeren Antriebs-Hohlwelle angeordnet, insbesondere gelagert ist.

10. Instrumentenanordnung nach einem der Ansprüche 5 bis 9, **dadurch gekennzeichnet, dass** wenigstens ein Drehantrieb der Antriebseinheit koaxial, insbesondere fluchtend oder parallel versetzt, oder winkelig, insbesondere rechtwinkelig, zu seiner Antriebswelle angeordnet ist.

11. Instrumentenanordnung nach einem der Ansprüche 5 bis 10, **dadurch gekennzeichnet, dass** das Koppelteil der Antriebseinheit axial verschieblich, insbesondere vorgespannt, an der Antriebswelle angeordnet ist.

12. Instrumentenanordnung nach einem der Ansprüche 5 bis 11, **dadurch gekennzeichnet, dass** das Instrument wenigstens eine innere Antriebswelle, insbesondere Antriebs-Hohlwelle, aufweist, die konzentrisch in einer äußeren Antriebs-Hohlwelle angeordnet, insbesondere gelagert ist.

13. Instrumentenanordnung nach einem der Ansprüche 5 bis 12, **dadurch gekennzeichnet, dass** die Antriebswelle des Instruments koaxial, insbesondere fluchtend oder parallel versetzt, oder winkelig, insbesondere rechtwinkelig, zu dem Instrumentenschaft angeordnet ist.

14. Instrumentenanordnung nach einem der Ansprüche 5 bis 13, **dadurch gekennzeichnet, dass** das Instrument ein Umsetzungsgetriebe zur Umsetzung einer Rotation einer Antriebswelle in eine Translation eines Zug- und/oder Schubmittels aufweist, wobei das Umsetzungsgetriebe insbesondere eine Kulissenführung (23, 24, 25, 37, 38) aufweist und/oder insbesondere in einer antriebseinheitszu- oder -abgewandten Hälfte des Instrumentenschaftes angeordnet ist.

15. Chirurgierobotersystem mit:
einem Roboter; und
einer, insbesondere lösbar, an dem Roboter befestigten Instrumentenanordnung nach einem der Ansprüche 5 bis 14.

16. Verfahren zum Umhüllen eines Roboters oder einer Antriebseinheit für eine Instrumentenanordnung nach einem der Ansprüche 5 bis 14 mit wenigstens einem Drehantrieb (7, 8, 9) mit einer Antriebswelle, insbesondere Antriebs-Hohlwelle (10, 13, 15; 106, 107, 108), mit einem Koppelteil (31, 32, 33; 200, 201, 202; 300, 301, 302) zur Koppelung mit einer Antriebswelle des Instruments mittels einer Hülle nach Anspruch 1,
**dadurch gekennzeichnet, dass**
die mit dem Blindstopfen (502) versehene Innendurchführung durch eine Hohlwelle und die Austrittsöffnung der Hülle durchgeführt, anschließend entfernt und der Abschlussring (503) an dem Umfangsbereich der Innendurchführung befestigt wird, der durch das Entfernen des Blindstopfens frei geworden ist.

## Claims

1. A sheath with a sleeve-like inner passage for guiding
through a hollow shaft of a robot or a drive unit for an instrument assembly according to any one of the claims 5 to 14, comprising at least one rotary drive (7, 8, 9) with a drive shaft, in particular a hollow drive shaft (10, 13, 15; 106, 107, 108), with a coupling part (31, 32, 33; 200, 201, 202; 300, 301, 302) for coupling with a drive shaft of the instrument and
through an outlet opening (507) of the sheath,
**characterized in that**
the inner passage comprises a blind plug (502), which comprises a tubular circumferential surface, which blind plug is attached to one end of the inner passage and which covers a circumferential portion of the inner passage, and a cap ring (503) for fastening to the outer circumferential area of the inner passage, which is released by removing the blind plug.

2. An instrument interface for an instrument assembly according to any one of the claims 5 to 14, **characterized by** a sheath (5, 501) according to claim 1, for ensheathing the drive unit of the instrument assembly.

3. The instrument interface according to claim 2, **characterized in that** the sheath (5, 501) comprises an inner passage (27) for an auxiliary instrument introduced through the instrument and the drive unit of the instrument assembly.

4. The instrument interface according to any one of the claims 2 to 3, **characterized by** at least one rotatable, in particular conical, intermediate element (19, 20, 21; 206, 207, 208) for coupling with the coupling part of a drive shaft of the drive unit and/or of the instrument in a friction-fitting or form-fitting manner.

5. An instrument assembly for being attached, in particular detachably, to the robot of a robotic surgery system according to claim 15, comprising:
a drive unit (2; 102) comprising at least one rotary drive (7, 8, 9) with a drive shaft, in particular a hollow drive shaft (10, 13, 15; 106, 107, 108), with a coupling part (31, 32, 33; 200, 201, 202; 300, 301, 302) for coupling with a drive shaft of the instrument;
an instrument (1; 100; 400) with an instrument shaft (3) and at least one drive shaft, in particular a hollow drive shaft (16, 17, 18; 109, 110, 111; 404, 405, 406), with a coupling part (34, 35, 36; 203, 204, 205; 303, 304, 305) for coupling with a drive shaft of the drive unit, which is releasably connected to the drive unit; and
an instrument interface (5, 500) according to any one of the claims 2 to 4, which is arranged between the drive unit and the instrument.

6. The instrument assembly according to claim 5, **characterized by** an auxiliary instrument (504) and/or a drive means, which is introduced, in particular releasably, through the instrument of the instrument assembly, for operating an end effector.

7. The instrument assembly according to any one of the claims 5 to 6, **characterized in that** at least one drive shaft (10, 13, 15; 106, 107, 108) of the drive unit and a drive shaft (16, 17, 18; 109, 110, 111; 404, 405, 406) of the instrument are arranged coaxially, in particular such that they are aligned.

8. The instrument assembly according to any one of the claims 5 to 7, **characterized in that** a coupling part (300, 301, 302) of a drive shaft of the drive unit and a coupling part (303, 304, 305) of a drive shaft of the instrument are magnetically coupled to each other.

9. The instrument assembly according to any one of the claims 5 to 8, **characterized in that** the drive unit comprises at least one inner drive shaft, in particular a hollow drive shaft, which is arranged concentrically within an outer hollow drive shaft, in particular supported therein.

10. The instrument assembly according to any one of the claims 5 to 9, **characterized in that** at least one rotary drive of the drive unit is arranged coaxially, in particular such that it is aligned, or with a parallel offset, or angled, in particular at right angle, with respect to its drive shaft.

11. The instrument assembly according to any one of the claims 5 to 10, **characterized in that** the coupling part of the drive unit is arranged axially displaceably, in particular with a bias, on the drive shaft.

12. The instrument assembly according to any one of the claims 5 to 11, **characterized in that** the instrument comprises at least one inner drive shaft, in particular a hollow drive shaft, which is arranged concentrically within an outer hollow drive shaft, in particular supported therein.

13. The instrument assembly according to any one of the claims 5 to 12, **characterized in that** the drive shaft of the instrument is arranged coaxially, in particular such that it is aligned, or with a parallel offset, or angled, in particular at right angle, with respect to the instrument shaft.

14. The instrument assembly according to any one of the claims 5 to 13, **characterized in that** the instrument comprises a conversion transmission for converting a rotation of a drive shaft into a translation of a tensile and/or a thrust means, wherein the conversion transmission in particular comprises a sliding sheath (23, 24, 25, 37, 38) and/or is in particular arranged in one half of the instrument shaft facing the drive unit or facing away from the drive unit.

15. A robotic surgery system, comprising:
a robot, and
an instrument assembly according to any one of the claims 5 to 14, in particular one that is detachably fastened to the robot.

16. A method of ensheathing a robot or a drive unit for an instrument assembly according to any one of the claims 5 to 14, comprising at least one rotary drive (7, 8, 9) with a drive shaft, in particular a hollow drive shaft (10, 13, 15; 106, 107, 108), with a coupling part (31, 32, 33; 200, 201, 202; 300, 301, 302) for coupling with a drive shaft of the instrument by means of a sheath according to claim 1,
**characterized in that**
the inner passage provided with the blind plug (502) is passed through a hollow shaft and the outlet opening of the sheath, thereafter the blind plug is removed and the cap ring (503) is fixed at the circumferential area of the inner passage, which has become accessible through the removal of the blind plug.

## Revendications

1. Enveloppe avec un passage intérieur tubulaire pour le passage
à travers un arbre creux d'un robot ou d'une unité d'entraînement pour un agencement d'instrument selon l'une quelconque des revendications 5 à 14 avec au moins un entraînement rotatif (7, 8, 9) avec un arbre d'entraînement, en particulier arbre creux d'entraînement (10, 13, 15 ; 106, 107, 108), avec une partie d'accouplement (31, 32, 33 ; 200, 201, 202 ; 300, 301, 302) pour l'accouplement avec un arbre d'entraînement de l'instrument et
à travers une ouverture de sortie (507) de l'enveloppe,
**caractérisée en ce que**
le passage intérieur présente un bouchon obturateur (502), lequel présente une surface d'enveloppe tubulaire, est fixé à une extrémité du passage intérieur et couvre une zone périphérique du passage intérieur, et une bague de fermeture (503) pour la fixation à la zone périphérique extérieure du passage intérieur, qui est libérée par retrait du bouchon obturateur.

2. Interface d'instrument pour un agencement d'instrument selon l'une quelconque des revendications 5 à 14, **caractérisée par** une enveloppe (5, 501) selon la revendication 1, pour l'enveloppement de l'unité d'entraînement de l'agencement d'instrument.

3. Interface d'instrument selon la revendication 2, **caractérisée en ce que** l'enveloppe (5, 501) présente un passage intérieur (27) pour un instrument supplémentaire introduit à travers l'instrument et l'unité d'entraînement de l'agencement d'instrument.

4. Interface d'instrument selon l'une quelconque des revendications 2 à 3, **caractérisée par** au moins un élément intermédiaire (19, 20, 21 ; 206, 207, 208) rotatif, en particulier conique, pour l'accouplement par friction ou correspondance de forme avec la partie d'accouplement d'un arbre d'entraînement de l'unité d'entraînement et/ou de l'instrument.

5. Agencement d'instrument pour la fixation, en particulier amovible, au niveau du robot d'un système robotisé chirurgical selon la revendication 15, avec :
une unité d'entraînement (2 ; 102) avec au moins un entraînement rotatif (7, 8, 9) avec un arbre d'entraînement, en particulier arbre creux d'entraînement (10, 13, 15 ; 106, 107, 108), avec une partie d'accouplement (31, 32, 33 ; 200, 201, 202 ; 300, 301, 302) pour l'accouplement avec un arbre d'entraînement de l'instrument ;
un instrument (1 ; 100 ; 400) avec un arbre d'instrument (3) et au moins un arbre d'entraînement, en particulier arbre creux d'entraînement (16, 17, 18 ; 109, 110, 111 ; 404, 405, 406), avec une partie d'accouplement (34, 35, 36 ; 203, 204, 205 ; 303, 304, 305) pour l'accouplement avec un arbre d'entraînement de l'unité d'entraînement, qui est reliée de manière amovible à l'unité d'entraînement ; et
une interface d'instrument (5, 500) selon l'une quelconque des revendications 2 à 4, qui est agencée entre l'unité d'entraînement et l'instrument.

6. Agencement d'instrument selon la revendication 5, **caractérisé par** un instrument supplémentaire (504) et/ou un moyen d'entraînement pour l'actionnement d'un effecteur final, lequel est introduit, en particulier de manière amovible, à travers l'instrument de l'agencement d'instrument.

7. Agencement d'instrument selon l'une quelconque des revendications 5 à 6, **caractérisé en ce qu'**au moins un arbre d'entraînement (10, 13, 15 ; 106, 107, 108) de l'unité d'entraînement et un arbre d'entraînement (16, 17, 18 ; 109, 110, 111 ; 404, 405, 406) de l'instrument sont agencés de manière coaxiale, en particulier alignée.

8. Agencement d'instrument selon l'une quelconque des revendications 5 à 7, **caractérisé en ce qu'**une partie d'accouplement (300, 301, 302) d'un arbre d'entraînement de l'unité d'entraînement et une partie d'accouplement (303, 304, 305) d'un arbre d'entraînement de l'instrument sont couplées magnétiquement l'une à l'autre.

9. Agencement d'instrument selon l'une quelconque des revendications 5 à 8, **caractérisé en ce que** l'unité d'entraînement présente au moins un arbre d'entraînement intérieur, en particulier arbre creux d'entraînement, qui est agencé, en particulier logé, de manière concentrique dans un arbre creux d'entrainement extérieur.

10. Agencement d'instrument selon l'une quelconque des revendications 5 à 9, **caractérisé en ce qu'**au moins un entraînement rotatif de l'unité d'entraînement est agencé de manière coaxiale, en particulier alignée ou parallèlement décalée, ou angulaire, en particulier à angle droit, par rapport à son arbre d'entrainement.

11. Agencement d'instrument selon l'une quelconque des revendications 5 à 10, **caractérisé en ce que** la partie d'accouplement de l'unité d'entraînement est agencée de manière axialement mobile, en particulier précontrainte, au niveau de l'arbre d'entrainement.

12. Agencement d'instrument selon l'une quelconque des revendications 5 à 11, **caractérisé en ce que** l'instrument présente au moins un arbre d'entraînement intérieur, en particulier arbre creux d'entraînement, qui est agencé, en particulier logé, de manière concentrique dans un arbre creux d'entraînement extérieur.

13. Agencement d'instrument selon l'une quelconque des revendications 5 à 12, **caractérisé en ce que** l'arbre d'entraînement de l'instrument est agencé de manière coaxiale, en particulier alignée ou parallèlement décalée, ou angulaire, en particulier à angle droit, par rapport à l'arbre d'entrainement.

14. Agencement d'instrument selon l'une quelconque des revendications 5 à 13, **caractérisé en ce que** l'instrument présente une transmission de conversion pour la conversion d'une rotation d'un arbre d'entraînement en une translation d'un moyen de traction et/ou de poussée, dans lequel la transmission de conversion présente en particulier un guide de coulisse (23, 24, 25, 37, 38) et/ou est agencé en particulier dans une moitié de l'arbre d'instrument tournée vers ou opposée à l'unité d'entraînement.

15. Système robotisé chirurgical avec :
un robot ; et
un agencement d'instrument fixé au robot, en particulier de manière amovible, selon l'une quelconque des revendications 5 à 14.

16. Procédé d'enveloppement d'un robot ou d'une unité d'entraînement pour un agencement d'instrument selon l'une quelconque des revendications 5 à 14 avec au moins un entraînement rotatif (7, 8, 9) avec un arbre d'entraînement, en particulier arbre creux d'entraînement (10, 13, 15 ; 106, 107, 108) avec une partie d'accouplement (31, 32, 33 ; 200, 201, 202 ; 300, 301, 302) pour l'accouplement avec un arbre d'entraînement de l'instrument au moyen d'une enveloppe selon la revendication 1,
**caractérisé en ce que**
le passage intérieur doté du bouchon obturateur (502) est guidé à travers un arbre creux et l'ouverture de sortie de l'enveloppe, puis le bouchon obturateur est retiré et la bague de fermeture (503) est fixée à la zone périphérique du passage intérieur qui est libérée par le retrait du bouchon obturateur.
